# FASCICULE DE BREVET EUROPEEN

(11) **EP 4 027 874 B1**
(45) Date de publication et mention de la délivrance du brevet: **02.08.2023**
(21) Numéro de dépôt: 20768061.2
(22) Date de dépôt: 11.09.2020
(51) Int. Cl.: A61B 5/024, A61B 5/08

(54) **PROCÉDÉ DE DÉTERMINATION DU TAUX RESPIRATOIRE**
VERFAHREN ZUR BESTIMMUNG DER ATEMFREQUENZ
METHOD FOR DETERMINING RESPIRATORY RATE

(30) Priorité: 13.09.2019 FR 1910162
(43) Date de publication de la demande: 20.07.2022
(73) Titulaire: Sensoria Analytics, 06140 Vence (FR)
(72) Inventeur: ARIDHI, Slaheddine, 06140 Vence (FR)
(74) Mandataire: Hautier IP
(86) Numéro de dépôt international: PCT/EP2020/075569
(87) Numéro de publication internationale: WO 2021/048422

(56) Documents cités:
- WO-A1-2013/179018
- WO-A1-2015/107268
- WALTER KARLEN ET AL: "Multiparameter Respiratory Rate Estimation From the Photoplethysmogram", IEEE TRANSACTIONS ON BIOMEDICAL ENGINEERING, IEEE SERVICE CENTER, PISCATAWAY, NJ, USA, vol. 60, no. 7, 1 juillet 2013 (2013-07-01), pages 1946-1953, XP011515762, ISSN: 0018-9294, DOI: 10.1109/TBME.2013.2246160

## Description

### DOMAINE TECHNIQUE DE L'INVENTION

La présente invention concerne le domaine des dispositifs de collecte de données médicales, en particulier des procédés de mesure d'un taux respiratoire chez un utilisateur.

### ETAT DE LA TECHNIQUE

Il existe de nombreuses solutions dans l'art antérieur visant à déterminer le taux respiratoire d'un utilisateur. Par exemple, des solutions utilisent la mesure du déplacement de la cage thoracique, d'autre la pression de l'air en sortie des voies respiratoires.

Les documents suivants concernent des solutions pour estimer une fréquence respiratoire : WO2013179018, WO2015107268, WALTER KARLEN ET AL, "Multiparameter Respiratory Rate Estimation From the Photoplethysmogram", IEEE TRANSACTIONS ON BIOMEDICAL ENGINEERING, IEEE SERVICE CENTER, PISCATAWAY, NJ, USA, (20130701), vol. 60, no. 7, doi:10.1109/TBME.2013.2246160.

Une des principales problématiques des solutions de l'art antérieur se trouve dans l'encombrement qu'entraîne ce type de solutions. Bien souvent l'utilisateur doit porter sur lui un équipement portable pas facile à ajuster ou un équipement non-portable et encombrant.

De nouvelles solutions ont été imaginées, mais sans succès, en se basant sur le rythme cardiaque pour en déduire le rythme respiratoire. Ces solutions ont généralement conduit à un manque de reproductibilité, à une fiabilité très faible ou encore à des erreurs importantes du taux respiratoire ainsi déterminé.

La présente invention se propose de solutionner ces diverses problématiques.

### RÉSUMÉ DE L'INVENTION

La présente invention concerne un procédé de détermination du taux respiratoire R d'au moins un utilisateur comprenant au moins les étapes suivantes mises en oeuvre par au moins une unité de traitement de données comprenant par exemple au moins l'un parmi un processeur et un ordinateur :
a. De préférence, acquisition d'au moins un photoplethysmogramme S(t) dudit utilisateur, l'étape d'acquisition dudit photoplethysmogramme S(t) comprenant au moins l'une parmi les étapes suivantes :
   i. Mesure, de préférence à une fréquence supérieure ou égale à 50Hz, dudit au moins un photoplethysmogramme S(t) par un dispositif de mesure, de préférence porté ou destiné à être porté par ledit au moins un utilisateur ;
   ii. Réception depuis au moins une base de données dudit au moins un photoplethysmogramme S(t) ;
b. Estimation de la fréquence cardiaque Fc à partir dudit au moins un photoplethysmogramme S(t) acquis dudit au moins un utilisateur ;
c. Obtention d'au moins trois signaux (S1, ...S7) distincts les uns des autres, chaque signal obtenu (S1, ...S7) étant fonction dudit au moins un photoplethysmogramme S(t) acquis ; Chacun de ces signaux peut être désigné signal primaire.
d. Génération d'un signal S8 à partir desdits au moins trois signaux (S1, ..., S7) obtenus (S1, ...S7). Ce signal peut être désigné signal complexe.
e. Traitement algorithmique de chacun des au moins trois signaux obtenus (S1, ...S7) et du signal S8 par au moins deux algorithmes Ai et Aj de sorte à obtenir au moins deux taux respiratoires estimés, respectivement Rx.Ai et Rx.Aj, pour chaque signal Sx traité respectivement par l'algorithme Ai et par l'algorithme Aj, chaque signal Sx étant pris parmi lesdits au moins trois signaux obtenus (S1, ..., S7) et le signal S8; les au moins deux algorithmes Ai et Aj étant distincts l'un de l'autre ;
f. Calcul d'au moins deux taux respiratoires intermédiaires rm et rn à partir des taux respiratoires estimés (Rx.Ai, Rx.Aj) ;
g. Détermination du taux respiratoire R en calculant la médiane desdits au moins deux taux respiratoires intermédiaires rm et rn.

La présente invention permet de déterminer de manière simple, rapide et fiable le taux respiratoire d'un utilisateur à partir de la mesure d'un photoplethysmogramme.

La présente invention permet d'utiliser un dispositif de mesure simple et ne requiert pas une haute précision dans la mesure du photoplethysmogramme.

La présente invention permet ainsi une économie de ressource matérielle et de calcul pour la détermination d'un paramètre physiologique à partir d'une simple mesure d'un photoplethysmogramme sur une durée très courte.

De préférence, le temps d'acquisition du photoplethysmogramme est supérieur à 30 secondes, de préférence à 1 minute et avantageusement à 2 minutes.

L'exploitation de la diversité des signaux respiratoires que l'on peut extraire à partir d'un signal pléthysmographie et l'utilisation de plusieurs approches algorithmiques permet d'engendrer une richesse d'information permettant ainsi d'estimer le taux respiratoire de façon fiable tout en minimisant l'erreur moyenne absolue appelée EMA.

Avantageusement, la présente invention permet d'obtenir une valeur d'un taux respiratoire d'un utilisateur à partir d'une mesure pouvant être bruitée.

Avantageusement, la présente invention permet d'obtenir une valeur d'un taux respiratoire d'un utilisateur à partir d'un dispositif de mesure pouvant être bon marché.

Avantageusement, la présente invention permet d'obtenir une valeur fiable d'un taux respiratoire d'un utilisateur.

La présente invention concerne également un procédé de détermination du taux respiratoire R d'au moins un utilisateur comprenant au moins les étapes suivantes :
a. Acquisition d'au moins un photoplethysmogramme S(t) dudit utilisateur, de préférence à une fréquence supérieure ou égale à 50Hz ;
b. Filtrage dudit photoplethysmogramme, de préférence par un filtre passe-bande, de préférence compris entre 0.5Hz et 4Hz, de sorte à générer un premier photoplethysmogramme filtré ;
c. Estimation de la fréquence cardiaque Fc à partir du photoplethysmogramme filtré, de préférence en identifiant la valeur maximale de la densité spectrale de puissance du photoplethysmogramme filtré ;
d. Filtrage du premier photoplethysmogramme filtré entre Fc/20 et 4Hz de sorte à générer un deuxième photoplethysmogramme filtré ;
e. Obtention d'au moins trois signaux distincts les uns des autres pris parmi les signaux suivants S1, S2, S3, S4, S5, S6 et S7 :
   i. un signal S1 obtenu par
      - Application d'un filtre passe-bande entre Fc/20 et Fc/2 au deuxième photoplethysmogramme filtré.
   ii. un signal S2 obtenu par :
      - détermination des sommets des battements de l'enveloppe supérieure du deuxième photoplethysmogramme filtré,
      - puis par filtrage du signal des sommets des battements de l'enveloppe supérieure par un filtre passe-bande entre Fc/20 et Fc/2.
   iii. un signal S3 obtenu par :
      - détermination des vallées des battements de l'enveloppe inférieure du deuxième photoplethysmogramme filtré ;
      - puis par filtrage du signal des vallées des battements de l'enveloppe inférieure par un filtre passe-bande entre Fc/20 et Fc/2 ;
         iv. un signal S4 obtenu par :
      - Génération d'une onde de vélocité du pouls VPG en effectuant une dérivée de première ordre du photoplethysmogramme S(t) par rapport à la coordonnée temporelle dudit photoplethysmogramme S(t) ;
      - Détermination des positions Pi des sommets de l'onde de vélocité du pouls VPG ;
      - Obtention d'un signal de variation des points d'inflexion en reportant des valeurs dudit photoplethysmogramme S(t) aux positions Pi ;
      - Sur-échantillonnage du signal de variation des points d'inflexion jusqu'à une fréquence Fs prédéterminée, de préférence généralement équivalente à celle du signal S(t) ;
      - Filtrage en passe-bande entre les fréquences Fc/20 et Fc/2 du signal suréchantillonné ;
         v. un signal S5 obtenu par :
      - Localisation des points d'inflexion Pi dans une montée systolique d'un battement de coeur de l'utilisateur en identifiant la position des sommets de la dérivée de premier ordre du photoplethysmogramme S(t) par rapport à la coordonnée temporelle dudit photoplethysmogramme S(t)
      - Obtention d'un signal de variation des points d'inflexion Pi en reportant des valeurs de différences temporelles entre Pi et Pi-1;
      - Sur-échantillonnage de ce signal d'intervalles des points d'inflexion Pi jusqu'à une fréquence Fs prédéterminée, de préférence généralement du même ordre que S(t);
      - Filtrage en passe-bande entre les fréquences Fc/20 et Fc/2 du signal suréchantillonné ;
         vi. un signal S6 obtenu par :
      - détermination des variations d'amplitude du deuxième photoplethysmogramme filtré,
      - puis par filtrage par un filtre passe-bande entre Fc/20 et Fc/2 du signal de variations d'amplitude ;
         vii. un signal S7 obtenu par :
      - détermination des vallées des battements de l'enveloppe inférieure du deuxième photoplethysmogramme filtré,
      - suréchantillonnage du signal des vallées des battements de l'enveloppe inférieure jusqu'à une fréquence Fs prédéterminée, de préférence généralement du même ordre que la fréquence du signal S(t) ;
      - Dérivation au premier ordre selon la coordonnée temporelle du signal suréchantillonné ;
      - puis par filtrage du signal dérivé par un filtre passe-bande entre Fc/20 et Fc/2 ;
f. Génération d'un signal S8 à partir desdits au moins trois signaux obtenus, l'étape de génération du signal S8 comprenant les étapes suivantes :
   i. Calcul, pour chaque signal desdits au moins trois signaux obtenus, d'un signal d'autocorrélation normalisé ;
   ii. Calcul de la moyenne arithmétique des signaux d'autocorrélations normalisés;
g. Traitement algorithmique de chacun des au moins trois signaux obtenus et du signal S8 par au moins deux algorithmes Ai et Aj de sorte à obtenir au moins deux taux respiratoires estimés, respectivement Rx.Ai et Rx.Aj, pour chaque signal Sx traité respectivement par l'algorithme Ai et par l'algorithme Aj, chaque signal Sx étant pris parmi lesdits au moins trois signaux obtenus et le signal S8 ; les au moins deux algorithmes Ai et Aj étant distincts l'un de l'autre et étant pris parmi au moins les algorithmes A1, A2, A3 et A4 suivants :
   i. un algorithme A1 comprenant au moins les étapes suivantes appliquées au signal Sx :
      - Collecte des points de mesure correspondant à un croisement des abscisses par le signal considéré, soit Sx(t) = 0 ;
      - Calcul de l'intervalle moyen Tm entre chacun des points de traversé des abscisses ;
      - Calcul de Rx.A1 avec Rx.A1 = 60/(2*Tm) ;
   ii. un algorithme A2 comprenant au moins les étapes suivantes appliquées au signal Sx :
      - Collecte des points de mesure correspondant au sommet des ondulations du signal considéré ;
      - Calcul de l'intervalle moyen Ts entre chacun des points des sommets ;
      - Calcul de Rx.A2 avec Rx.A2 = 60/(Ts) ;
   iii. un algorithme A3 comprenant au moins les étapes suivantes appliquées au signal Sx :
      - Détermination de la densité spectrale de correntropie en calculant la transformée de Fourrier de l'autocorrentropie centrée du signal considéré ;
      - Extraction de Rx.A3 en déterminant le sommet de la densité spectrale de correntropie sur une plage fréquentielle prédéterminée, de
      préférence entre max(Fc/20, 0.05) et min(Fc/2,1) où 0.05Hz et 1 Hz correspondent respectivement à des taux respiratoires de 3 et 60 respirations par minute ;
   iv. un algorithme A4 comprenant au moins les étapes suivantes appliquées au signal Sx :
      - Calcul du signal d'autocorrélation du signal considéré ;
      - Collecte des points de mesure correspondant au sommet des ondulations du signal d'autocorrélation du signal considéré ;
      - Calcul de l'intervalle moyen Tas entre chacun des points des sommets ;
      - Calcul de Rx.A4 avec Rx.A4 = 60/(Tas) ;

   h. Calcul d'au moins deux taux respiratoires intermédiaires rm et rn pris parmi au moins les taux respiratoires intermédiaires r1, r2, r3 et r4 suivants :
      i. r1 est égal à la valeur médiane des taux respiratoires estimés Rx.Ay d'un signal Sx, Sx étant pris parmi au moins les signaux obtenus ;
      ii. r2 correspond au sommet de l'histogramme formé par les taux respiratoires estimés Rx.Ay de chaque signal Sx considéré pris parmi les signaux obtenus traités au travers de chaque algorithme Ay considéré pris parmi A1 à A4 ;
      iii. r3 est égal à la valeur médiane des taux respiratoires estimés Rx.A3 pour chaque signal Sx considéré pris parmi les signaux obtenus traité par l'algorithme A3 ;
      iv. r4 est égal à la valeur médiane des taux respiratoires estimés R8.Ay du signal S8 traité au travers de chaque algorithme Ay considéré pris parmi A1 à A4 ;
   i. Détermination du taux respiratoire R en calculant la médiane desdits au moins deux taux respiratoires intermédiaires.

La présente invention concerne aussi un produit programme d'ordinateur, de préférence enregistré sur un support non transitoire, comprenant des instructions, qui lorsqu'elles sont effectuées par au moins l'un parmi un processeur et un ordinateur, font que l'au moins un parmi le processeur et l'ordinateur, exécute le procédé selon l'une quelconque des revendications précédentes.

La présente invention concerne enfin un dispositif de mesure d'un photoplethysmogramme S(t) d'un utilisateur connecté à au moins une unité de traitement de données comprenant au moins une mémoire non transitoire comprenant un produit programme d'ordinateur selon la revendication précédente, de préférence, le dispositif de mesure comprend au moins un capteur apte à mesurer un photoplethysmogramme.

La présente invention concerne également un procédé de détermination du taux respiratoire R d'au moins un utilisateur comprenant au moins les étapes suivantes mises en oeuvre par au moins une unité de traitement de données comprenant par exemple au moins l'un parmi un processeur et un ordinateur :
a. De préférence, acquisition d'au moins un signal fonction du rythme respiratoire S(t) dudit utilisateur, l'étape d'acquisition dudit signal fonction du rythme respiratoire S(t) comprenant au moins l'une parmi les étapes suivantes :
   i. Mesure, de préférence à une fréquence supérieure ou égale à 50Hz, dudit au moins un signal fonction du rythme respiratoire S(t) par un dispositif de mesure, de préférence porté ou destiné à être porté par ledit au moins un utilisateur ;
   ii. Réception depuis au moins une base de données dudit au moins un signal fonction du rythme respiratoire S(t) ;
b. Estimation de la fréquence cardiaque Fc à partir dudit au moins un signal fonction du rythme respiratoire S(t) acquis dudit au moins un utilisateur ;
c. Obtention d'au moins trois signaux (S1, ...S7) distincts les uns des autres, chaque signal obtenu (S1, ...S7) étant fonction dudit au moins un signal fonction du rythme respiratoire S(t) acquis ; Chacun de ces signaux peut être désigné signal primaire.
d. Génération d'un signal S8 à partir desdits au moins trois signaux (S1, ..., S7) obtenus (S1, ...S7). Ce signal peut être désigné signal complexe.
e. Traitement algorithmique de chacun des au moins trois signaux obtenus (S1, ...S7) et du signal S8 par au moins deux algorithmes Ai et Aj de sorte à obtenir au moins deux taux respiratoires estimés, respectivement Rx.Ai et Rx.Aj, pour chaque signal Sx traité respectivement par l'algorithme Ai et par l'algorithme Aj, chaque signal Sx étant pris parmi lesdits au moins trois signaux obtenus (S1, ..., S7) et le signal S8; les au moins deux algorithmes Ai et Aj étant distincts l'un de l'autre ;
f. Calcul d'au moins deux taux respiratoires intermédiaires rm et rn à partir des taux respiratoires estimés (Rx.Ai, Rx.Aj) ;
g. Détermination du taux respiratoire R en calculant la médiane desdits au moins deux taux respiratoires intermédiaires rm et rn.

De préférence, le signal fonction du rythme respiratoire comprend ou est un photoplethysmogramme.

### BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La figure 1 représente un dispositif de mesure selon un mode de réalisation de la présente invention.
La figure 2 représente un dispositif de mesure selon un autre mode de réalisation de la présente invention.
La figure 3 représente un diagramme schématisant un mode de réalisation du procédé selon la présente invention.
La figure 4 représente un exemple de photoplethysmogramme S(t) d'un utilisateur.
La figure 5 représente le signal S1 selon un mode de réalisation de la présente invention.
La figure 6 représente le signal S2 selon un mode de réalisation de la présente invention.
La figure 7 représente le signal S3 selon un mode de réalisation de la présente invention.
La figure 8 représente le signal S4 selon un mode de réalisation de la présente invention.
La figure 9 représente le signal S5 selon un mode de réalisation de la présente invention.
La figure 10 représente le signal S6 selon un mode de réalisation de la présente invention.
La figure 11 représente le signal S7 selon un mode de réalisation de la présente invention.
La figure 12 représente le signal S8 selon un mode de réalisation de la présente invention.
La figure 13 représente le calcul d'un taux respiratoire intermédiaire r2 selon un mode de réalisation de la présente invention.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques. En particulier les dimensions ne sont pas représentatives de la réalité.

### DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement.

Selon un mode de réalisation, le procédé comprend, avant l'étape d'estimation de la fréquence cardiaque Fc, une étape de filtrage dudit photoplethysmogramme, de préférence par un filtre passe-bande, de préférence compris entre 0.5Hz et 4Hz, de sorte à générer un premier photoplethysmogramme filtré.

Selon un mode de réalisation, le procédé comprend, après l'étape d'estimation de la fréquence cardiaque Fc, une étape de filtrage additionnelle dudit premier photoplethysmogramme filtré entre Fc/20 et 4Hz de sorte à générer un deuxième photoplethysmogramme filtré

Selon un mode de réalisation, lesdits au moins trois signaux (S1, ..., S7) distincts obtenus sont pris parmi au moins les signaux suivants S1, S2, S3, S4, S5, S6 et S7 :
a. un signal S1 obtenu par :
   i. Application d'un filtre passe-bande entre Fc/20 et Fc/2 au deuxième photoplethysmogramme filtré.
b. un signal S2 obtenu par :
   i. détermination des sommets des battements de l'enveloppe supérieure du deuxième photoplethysmogramme filtré,
   ii. puis par filtrage du signal des sommets des battements de l'enveloppe supérieure par un filtre passe-bande entre Fc/20 et Fc/2.
c. un signal S3 obtenu par :
   i. détermination des vallées des battements de l'enveloppe inférieure du deuxième photoplethysmogramme filtré ;
   ii. puis par filtrage du signal des vallées des battements de l'enveloppe inférieure par un filtre passe-bande entre Fc/20 et Fc/2 ;
d. un signal S4 obtenu par :
   i. Génération d'une onde de vélocité du pouls VPG en effectuant une dérivée de première ordre du photoplethysmogramme S(t) par rapport à la coordonnée temporelle dudit photoplethysmogramme S(t) ;
   ii. Détermination des positions Pi des sommets de l'onde de vélocité du pouls VPG ;
   iii. Obtention d'un signal de variation des points d'inflexion en reportant des valeurs dudit photoplethysmogramme S(t) aux positions Pi ;
   iv. Sur-échantillonnage du signal de variation des points d'inflexion jusqu'à une fréquence Fs prédéterminée, de préférence généralement équivalente à celle du signal S(t) ;
   v. Filtrage en passe-bande entre les fréquences Fc/20 et Fc/2 du signal suréchantillonné ;
e. un signal S5 obtenu par :
   i. Localisation des points d'inflexion Pi dans une montée systolique d'un battement de coeur de l'utilisateur en identifiant la position des sommets de la dérivée de premier ordre du photoplethysmogramme S(t) par rapport à la coordonnée temporelle dudit photoplethysmogramme S(t)
   ii. Obtention d'un signal de variation des points d'inflexion Pi en reportant des valeurs de différences temporelles entre Pi et Pi-1;
   iii. Sur-échantillonnage de ce signal d'intervalles des points d'inflexion Pi jusqu'à une fréquence Fs prédéterminée, de préférence généralement du même ordre que S(t);
   iv. Filtrage en passe-bande entre les fréquences Fc/20 et Fc/2 du signal suréchantillonné ;
f. un signal S6 obtenu par :
   i. détermination des variations d'amplitude du deuxième photoplethysmogramme filtré,
   ii. puis par filtrage par un filtre passe-bande entre Fc/20 et Fc/2 du signal de variations d'amplitude ;
g. un signal S7 obtenu par :
   i. détermination des vallées des battements de l'enveloppe inférieure du deuxième photoplethysmogramme filtré,
   ii. suréchantillonnage du signal des vallées des battements de l'enveloppe inférieure jusqu'à une fréquence Fs prédéterminée, de préférence généralement du même ordre que la fréquence du signal S(t);
   iii. Dérivation au premier ordre selon la coordonnée temporelle du signal suréchantillonné ;
   iv. puis par filtrage du signal dérivé par un filtre passe-bande entre Fc/20 et Fc/2 ;

Selon un mode de réalisation, l'étape de génération d'un signal S8 à partir desdits au moins trois signaux obtenus (S1, ...S7) comprend au moins les étapes suivantes :
a. Calcul, pour chaque signal parmi lesdits au moins trois signaux obtenus (S1, ..., S7), d'un signal d'autocorrélation normalisé ;
b. Calcul de la moyenne arithmétique des signaux d'autocorrélations normalisés.

Selon un mode de réalisation, les au moins deux algorithmes Ai et Aj distincts l'un de l'autre sont pris parmi au moins les algorithmes A1, A2, A3 et A4 suivants :
a. un algorithme A1 comprenant au moins les étapes suivantes appliquées au signal Sx :
   i. Collecte des points de mesure correspondant à un croisement des abscisses par le signal considéré, soit Sx(t) = 0 ;
   ii. Calcul de l'intervalle moyen Tm entre chacun des points de traversé des abscisses ;
   iii. Calcul de Rx.A1 avec Rx.A1 = 60/(2*Tm) ;
b. un algorithme A2 comprenant au moins les étapes suivantes appliquées au signal Sx :
   i. Collecte des points de mesure correspondant au sommet des ondulations du signal considéré ;
   ii. Calcul de l'intervalle moyen Ts entre chacun des points des sommets ;
   iii. Calcul de Rx.A2 avec Rx.A2 = 60/(Ts) ;
c. un algorithme A3 comprenant au moins les étapes suivantes appliquées au signal Sx :
   i. Détermination de la densité spectrale de correntropie en calculant la transformée de Fourrier de l'autocorrentropie centrée du signal considéré ;
   ii. Extraction de Rx.A3 en déterminant le sommet de la densité spectrale de correntropie sur une plage fréquentielle prédéterminée, de préférence entre max(Fc/20, 0.05) et min(Fc/2,1) où 0.05Hz et 1 Hz correspondent respectivement à des taux respiratoires de 3 et 60 respirations par minute ;
d. un algorithme A4 comprenant au moins les étapes suivantes appliquées au signal Sx :
   i. Calcul du signal d'autocorrélation du signal considéré ;
   ii. Collecte des points de mesure correspondant au sommet des ondulations du signal d'autocorrélation du signal considéré ;
   iii. Calcul de l'intervalle moyen Tas entre chacun des points des sommets ;
   iv. Calcul de Rx.A4 avec Rx.A4 = 60/(Tas) ;

Selon un mode de réalisation, les au moins deux taux respiratoires intermédiaires rm et rn sont pris parmi au moins les taux respiratoires intermédiaires r1, r2, r3 et r4 suivants :
a. r1 est égal à la valeur médiane des taux respiratoires estimés Rx.Ay d'un signal Sx, Sx étant pris parmi au moins les signaux obtenus ;
b. r2 correspond au sommet de l'histogramme formé par les taux respiratoires estimés Rx.Ay de chaque signal Sx considéré pris parmi les signaux obtenus traités au travers de chaque algorithme Ay considéré pris parmi A1 à A4 ;
c. r3 est égal à la valeur médiane des taux respiratoires estimés Rx.A3 pour chaque signal Sx considéré pris parmi les signaux obtenus traité par l'algorithme A3 ;
d. r4 est égal à la valeur médiane des taux respiratoires estimés R8.Ay du signal S8 traité au travers de chaque algorithme Ay considéré pris parmi A1 à A4.

Selon un mode de réalisation, le procédé comprend une étape d'acquisition dudit au moins un photoplethysmogramme S(t) dudit utilisateur, de préférence à une fréquence supérieure ou égale à 50Hz.

Selon un mode de réalisation, l'étape d'acquisition dudit photoplethysmogramme S(t) comprend la mesure dudit au moins un photoplethysmogramme S(t) par un dispositif, de préférence porté ou destiné à être porté par ledit au moins un utilisateur.

Selon un mode de réalisation, l'étape d'obtention d'au moins trois signaux (S1, ..., S7) distincts les uns des autres comprend l'obtention d'au moins 4, de préférence d'au moins 5, avantageusement d'au moins 6 et avantageusement des 7 signaux distincts S1, S2, S3, S4, S5, S6 et S7.

Avantageusement, plus l'on considère de signaux respiratoires estimés, et meilleure est la probabilité d'estimation du taux respiratoire avec un minimum d'erreur.

Selon un mode de réalisation, l'étape de traitement algorithmique comprend l'utilisation d'au moins 3 et de préférence des 4 algorithmes distincts A1, A2, A3 et A4 appliqué à chacun des signaux obtenus (S1, ..., S7) et du signal S8.

Avantageusement, la diversité d'algorithme d'estimation du taux respiratoire permet de traiter toutes formes de signaux respiratoires (avec ou sans artefact) tout en offrant une convergence rapide vers le résultat recherché.

Selon un mode de réalisation, r1 = médiane( médiane ((R1.A1 , R1.A2 , R1.A3 , R1.A4)) , médiane((R2.A1 , R2.A2 , R2.A3 , R2.A4)) , médiane ((R3.A1 , R3.A2 , R3.A3 , R3.A4)) , médiane ((R4.A1 , R4.A2 , R4.A3 , R4.A4)) , médiane ((R5.A1 , R5.A2 , R5.A3 , R5.A4)) , médiane ((R6.A1 , R6.A2 , R6.A3 , R6.A4)) , médiane ((R7.A1 , R7.A2 , R7.A3 , R7.A4))).

Avantageusement, cette technique favorise les estimations qui se produisent autour de 50% du temps, d'où l'utilisation de la médiane.

Selon un mode de réalisation, r2 correspond au sommet de l'histogramme formé par les valeurs suivantes : R1.A1, R1.A2, R1.A3, R1.A4, R2.A1, R2.A2, R2.A3, R2.A4, R3.A1, R3.A2, R3.A3, R3.A4, R4.A1, R4.A2, R4.A3, R4.A4, R5.A1, R5.A2, R5.A3, R5.A4, R6.A1, R6.A2, R6.A3, R6.A4, R7.A1, R7.A2, R7.A3, R7.A4.

Avantageusement, cette technique favorise les estimations les plus fréquentes et rapprochées.

Selon un mode de réalisation, r3 = médiane(R1.A3 , R2.A3, R3.A3 , R4.A3 , R5.A3 , R6.A3 , R7.A3).

Avantageusement, cette technique favorise les estimations basées sur l'analyse fréquentielle des signaux.

Selon un mode de réalisation, r4 = médiane(R8.A1 , R8.A2 , R8.A3 , R8.A4).

Selon un mode de réalisation, R = médiane(r1 , r2 , r3 , r4).

Selon un mode de réalisation, S8 = (S1c+S2c+S3c+S4c+S5c+S6c+S7c)/7, avec les signaux Sxc correspondant au signal d'autocorrélation normalisé du signal Sx pris parmi S1, S2, S3, S4, S5, S6 et S7.

Avantageusement, l'utilisation des signaux d'autocorrélation présente plusieurs avantages : les ondulations et la périodicité intrinsèques aux signaux sont mises en valeur. En plus, les signaux sont naturellement alignés pour obtenir une combinaison efficace. La normalisation assure que tous les signaux dans la combinaison ont le même poids.

Selon un mode de réalisation, le dispositif selon la présente invention comprend un module de communication sans fil avec ladite unité de traitement de données.

Selon un mode de réalisation, le dispositif selon la présente invention comprend un module de communication filaire avec ladite unité de traitement de données.

Selon un mode de réalisation, le capteur apte à mesurer un photoplethysmogramme est un capteur pris parmi au moins : capteur optique, capteur électrique, capteur radar, capteur de force, capteur de pression, capteur de vibration, capteur audio, capteur séismique

Selon un mode de réalisation, le dispositif selon la présente invention comprend au moins un capteur du rythme respiratoire dudit utilisateur pris parmi au moins : capteur optique, capteur électrique, capteur radar, capteur de force, capteur de pression, capteur de vibration, capteur audio, capteur séismique...

La présente invention concerne un procédé de détermination du taux respiratoire R d'au moins un utilisateur. De manière particulièrement astucieuse, ce procédé utilise un photoplethysmogramme S(t) dudit utilisateur.

Ce photoplethysmogramme S(t) peut être obtenu de diverses manières. En particulier, et selon un mode de réalisation de la présente invention, il peut être obtenu par un dispositif de mesure comprenant au moins un capteur pris parmi au moins un capteur optique, électrique, radar, de force de pression, de vibration, audio ou encore séismique par exemple.

Selon un mode de réalisation, un tel dispositif de mesure comprend au moins un capteur apte à mesurer au moins un paramètre physiologique de l'utilisateur et de préférence un paramètre lié indirectement ou non à la respiration de l'utilisateur.

Par exemple, et de manière préférée, le dispositif de mesure peut comprendre au moins un capteur optique et au moins une source lumineuse. Le capteur optique peut fonctionner de deux manières, soit par transparence, c'est-à-dire qu'il reçoit les rayons lumineux émis par la source lumineuse et ayant traversé une partie du corps de l'utilisateur, un doigt par exemple, soit par réflexion, c'est-à-dire qu'il reçoit les rayons lumineux émis par la source lumineuse et ayant été réfléchis par une partie du corps de l'utilisateur, par son doigt par exemple. De tels dispositifs seront décrits par la suite.

De manière préférée, un dispositif de mesure selon la présente invention est connecté à au moins une unité de traitement de données au moins une mémoire non transitoire comprenant un produit programme d'ordinateur. De préférence, ce produit programme d'ordinateur comprend des instructions, qui lorsqu'elles sont effectuées par au moins l'un parmi un processeur et un ordinateur, font que l'au moins un parmi le processeur et l'ordinateur, exécute le procédé selon la présente invention.

Selon un mode de réalisation le dispositif de mesure est en communication filaire et/ou non filaire avec ladite unité de traitement de données.

Comme cela sera décrit par la suite, et selon un mode de réalisation préféré, le procédé de détermination du taux respiratoire R d'un utilisateur comprend au moins les étapes suivantes mises en oeuvre par au moins une unité de traitement de données :
a. Acquisition d'au moins un photoplethysmogramme S(t) dudit utilisateur de préférence via un dispositif de mesure et/ou via une base de données ;
b. Un premier filtrage du photoplethysmogramme S(t) peut alors être effectué ;
c. Puis, le photoplethysmogramme filtré Sf(t) va alors être traité de sorte à obtenir une pluralité de signaux, de préférence au moins trois signaux distincts, avantageusement au moins 4, de préférence au moins 5, avantageusement au moins 6 et de préférence au moins 7, pris parmi des signaux S1, S2, S3, S4, S5, S6 et S7 décrits par la suite ;
d. Ces signaux distincts vont alors être compilés de sorte à former un autre signal que nous nommerons S8 et qui sera décrit par la suite ;
e. Puis, un traitement algorithmique sera effectué sur chacun des signaux ainsi obtenu de sorte à extraire pour chaque signal au moins un taux respiratoires estimés Rx.Ay par algorithme appliqué, x représentant l'un des signaux traité et étant donc pris parmi au moins 1, 2, 3, 4, 5, 6, 7, 8, et y correspondant à l'algorithme utilisé tel que décrit par la suite ;
f. Puis, sur la base de ces taux respiratoires estimés Rx.Ay, on calcule une pluralité de taux respiratoire intermédiaires rx, de préférence r1, r2, r3 et r4 ;
g. Enfin, on calcule le taux respiratoire R de l'utilisateur en déterminant la médiane des taux respiratoire intermédiaires rx, tel que R = médiane (r1, r2, r3, r4).

Ainsi la présente invention permet de déterminer le taux respiratoire d'un utilisateur à partir d'un photoplethysmogramme S(t) pouvant être obtenu de diverses manières.

Un des nombreux avantages de la présente invention consiste dans le fait que le signal photopléthysmographique (PPG) représente le signal cardiaque modulé par le signal respiratoire. Ce signal PPG est très riche en information et la présente invention permet exploite cette richesse pour extraire un ou plusieurs signaux respiratoires sur lesquels des estimations de taux respiratoires sont ensuite opérées.

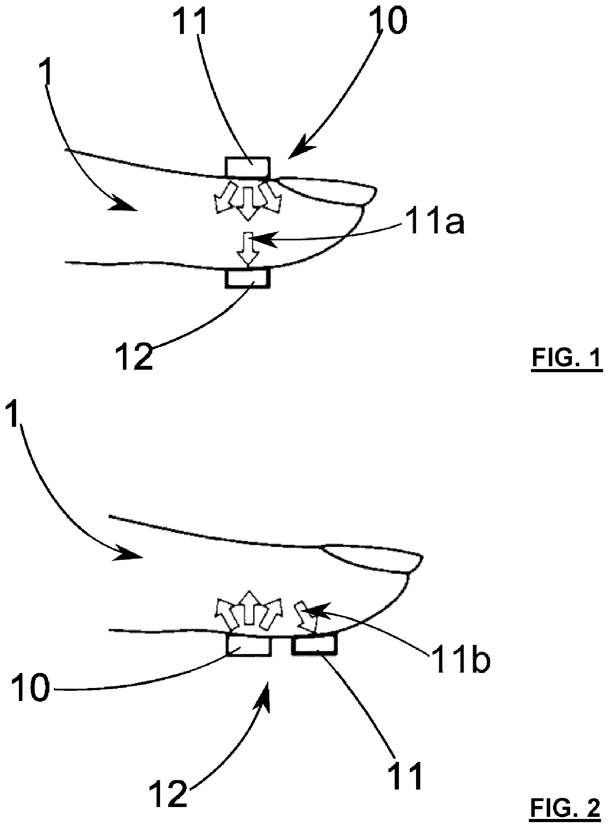

Nous allons à présent décrire un dispositif de mesure selon la présente invention au travers des figures 1 et 2.

Les figures 1 et 2 illustrent un dispositif de mesure 10 selon deux modes de réalisation de la présente invention. Ce dispositif 10 est disposé au contact d'un doigt 1 d'un utilisateur.

A titre d'information, la photopléthysmographie est la technique employée dans l'oxymétrie, qui est couramment utilisée pour mesurer la saturation du sang artériel (SpO2). La photopléthysmographie fournit des mesures non invasives, continues et en temps réel du signal représentant le contour de l'onde de pouls avec de la lumière infrarouge transmise à travers un doigt ou un orteil. Ce signal acquis est le volume numérique du pouls, une forme d'onde qui peut fournir par exemple des éléments sur l'état de santé d'une personne. Le calcul de l'onde de pouls a été démontré comme une technique fiable et reproductible pour déterminer indirectement les indices de la rigidité artérielle par exemple. La photopléthysmographie est donc une mesure utile non invasive de la dysfonction vasculaire et de la variabilité de la fréquence cardiaque.

Il existe principalement, mais non limitativement, deux modes de photopléthysmographie : la transmittance qui est communément utilisée dans l'oxymétrie et la réflectance qui est très utilisée dans la mesure de la fréquence cardiaque par les bracelets et montres connectées par exemple. Une source lumineuse transmet des rayons lumineux rouge et/ou infrarouge et un capteur optique capture la lumière transmise ou réfléchie.

On notera que pour la présente invention, un photoplethysmogramme S(t) s'entend d'un signal représentant les variations du volume numérique du pouls dans le temps et/ou du rythme respiratoire d'un utilisateur. Un tel signal peut aussi bien être acquis via un capteur optique que via un capteur électrique, un capteur radar, un capteur de force, un capteur de pression, un capteur de vibration, un capteur audio, ou encore capteur séismique. De préférence, on comprendra que selon la présente invention, le mot photoplethysmogramme sera utilisé également pour parler d'un plethysmogramme.

En particulier, on notera que via la présente invention, le rythme respiratoire d'un utilisateur peut être évalué par l'acquisition de données faite par un capteur optique, un capteur électrique, un capteur radar, un capteur de force, un capteur de pression, un capteur de vibration, un capteur audio, ou encore capteur séismique.

En effet, la présente invention s'applique à tout type de photoplethysmogrammes acquis par divers type de capteurs tels que ceux précédemment mentionnés, et/ou acquis depuis une base de données de photoplethysmogrammes.

De manière avantageuse, l'unité de traitement de données comprend au moins un processeur ou un ordinateur, et de préférence une mémoire non transitoire. De préférence, l'unité de traitement de données peut ainsi être un ordinateur, une montre intelligente, un téléphone intelligent, un dispositif médical, un serveur informatique, etc.... On notera qu'une unité de traitement de données peut également comprendre un dispositif de mesure d'un photoplethysmogramme.

Selon un mode de réalisation illustré en figure 1, le dispositif 10 est disposé au moins de part et d'autre du doigt 1 de l'utilisateur. Avantageusement, une source lumineuse 11 est disposée d'un côté du doigt 1 et un capteur optique 12 est disposé de l'autre côté du doigt 1 de sorte à pouvoir capter les rayons lumineux transmis 11a issus de la source lumineuse 11 et traversant le doigt 1 de l'utilisateur.

Selon un mode de réalisation illustré en figure 2, le dispositif 10 est disposé au moins d'un côté du doigt 1 de l'utilisateur. Avantageusement, une source lumineuse 11 est disposée d'un côté du doigt 1 et un capteur optique 12 est disposé du même côté du doigt 1 que la source lumineuse 11 de sorte à pouvoir capter les rayons lumineux réfléchis 11b issus de la source lumineuse 11 et étant réfléchis par le doigt 1 de l'utilisateur.

Dans chacun de ces deux modes de réalisation, le dispositif de mesure 10 est configuré pour mesurer un photoplethysmogramme S(t) 111 de l'utilisateur.

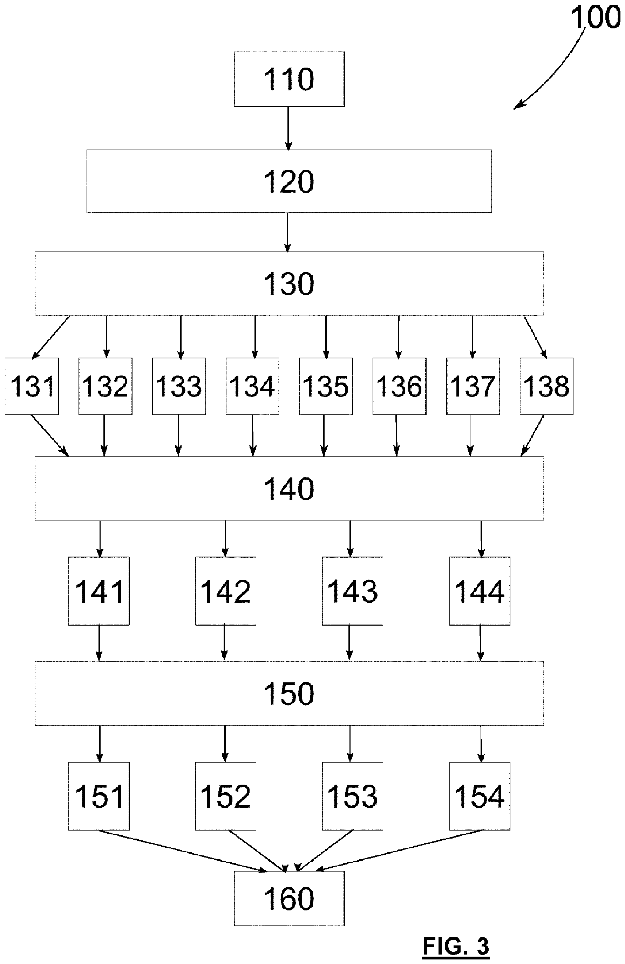

La figure 3 illustre un diagramme schématisant un mode de réalisation du procédé selon la présente invention.

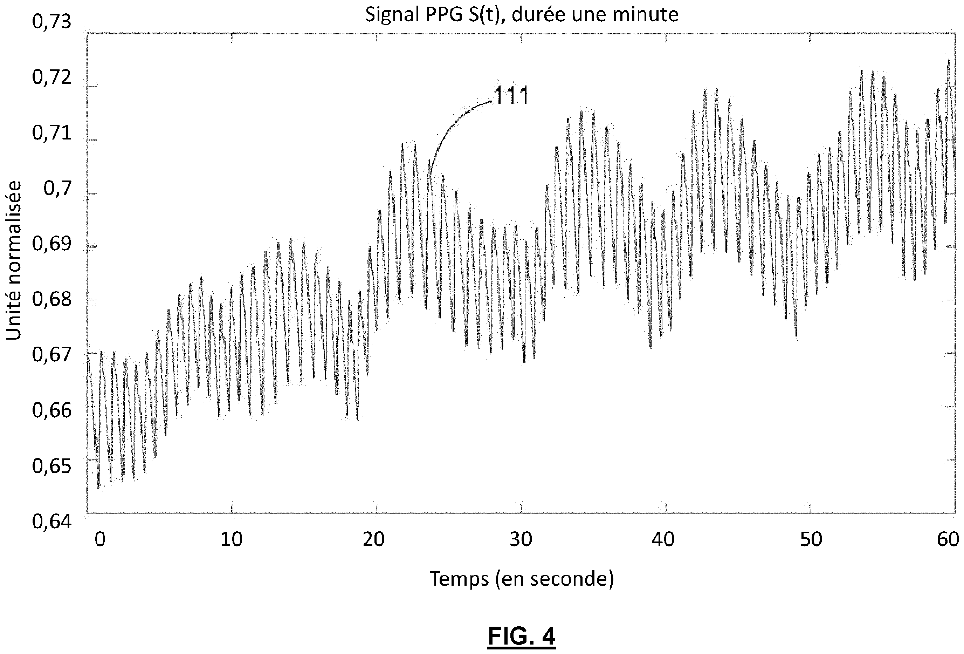

Ainsi, le procédé 100 de détermination du taux respiratoire R d'au moins un utilisateur comprenant au moins les étapes suivantes mises en oeuvre par au moins une unité de traitement de données :
a. Acquisition 110 d'au moins un photoplethysmogramme (appelé PPG) S(t) 111 dudit utilisateur, de préférence à une fréquence supérieure ou égale à 50Hz ; Le signal S(t) 111 est illustré en figure 4 selon un mode de réalisation de la présente invention ; Dans la présente invention on entend par acquisition d'au moins un PPG le fait d'obtenir par mesure un PPG et/ou le fait de recevoir des informations d'au moins une mesure déjà réalisée ; Ainsi, selon un mode de réalisation, l'étape d'acquisition 110 dudit au moins un photoplethysmogramme (appelé PPG) S(t) 111 dudit utilisateur est réalisé par réception depuis au moins une base de données dudit photoplethysmogramme S(t) 111 dudit utilisateur ;
b. Filtrage 120 de S(t), de préférence par un filtre passe-bande compris entre 0.5Hz et 4Hz, de sorte à générer un premier signal PPG filtré Sf(t) ;
c. Estimation 120 de la fréquence cardiaque Fc à partir de Sf(t), de préférence en identifiant la valeur maximale de la densité spectrale de puissance de Sf(t).
d. Filtrage de Sf(t) entre Fc/20 et 4Hz de sorte à générer un deuxième photoplethysmogramme filtré S'f(t) ;
e. Obtention 130 d'au moins trois signaux distincts les uns des autres Sa, Sb, Sc, de préférence d'au moins 4, de préférence d'au moins 5, avantageusement d'au moins 6 et de préférence d'au moins 7, lesdits signaux distincts étant pris parmi les signaux S1, S2, S3, S4, S5, S6 et S7 décrits ci-après ;
f. Calcul d'un huitième signal 138 dit S8 à partir desdits signaux obtenus ;
g. Traitement algorithmique 140 de chaque signaux dont S8 par au moins deux, de préférence au moins 3 et avantageusement 4 algorithmes A1, A2, A3 et A4 décrits par la suite, chaque algorithme étant configuré pour générer au moins un taux respiratoire estimé par signal traité de sorte à obtenir les taux respiratoires estimés Rx.Ay correspondant au taux respiratoire estimé à partir du signal Sx et de l'algorithme Ay.
h. Calcul 150 d'au moins deux, de préférence d'au moins 3 et avantageusement de 4 taux respiratoires intermédiaires r1, r2, r3 et r4 tels que décrits par la suite.
i. Détermination 160 du taux respiratoire R en réalisant la médiane desdits taux respiratoires intermédiaires.

Comme indiqué précédemment, et selon un mode de réalisation préféré, l'ensemble des étapes du présent procédé est réalisé par une unité de traitement de données du type ordinateur par exemple.

De manière avantageuse, la présente invention utilise une pluralité de traitements du signal PPG 111, ainsi qu'une pluralité d'algorithmes pour obtenir une valeur précise et fiable du taux respiratoire R de l'utilisateur.

Nous allons à présent détailler l'obtention 130 desdits signaux S1 à S7 et le calcul 138 du signal S8.

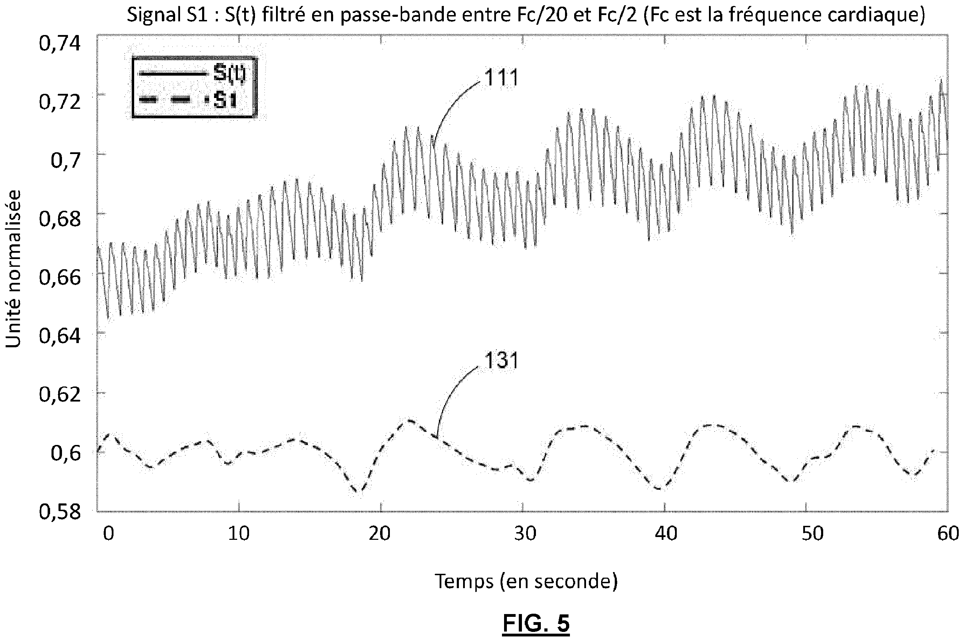

Le signal S1 131 est illustré en figure 5. Il est obtenu par l'application d'un filtre passe-bande entre Fc/20 et Fc/2 au deuxième photoplethysmogramme filtré S'f(t).

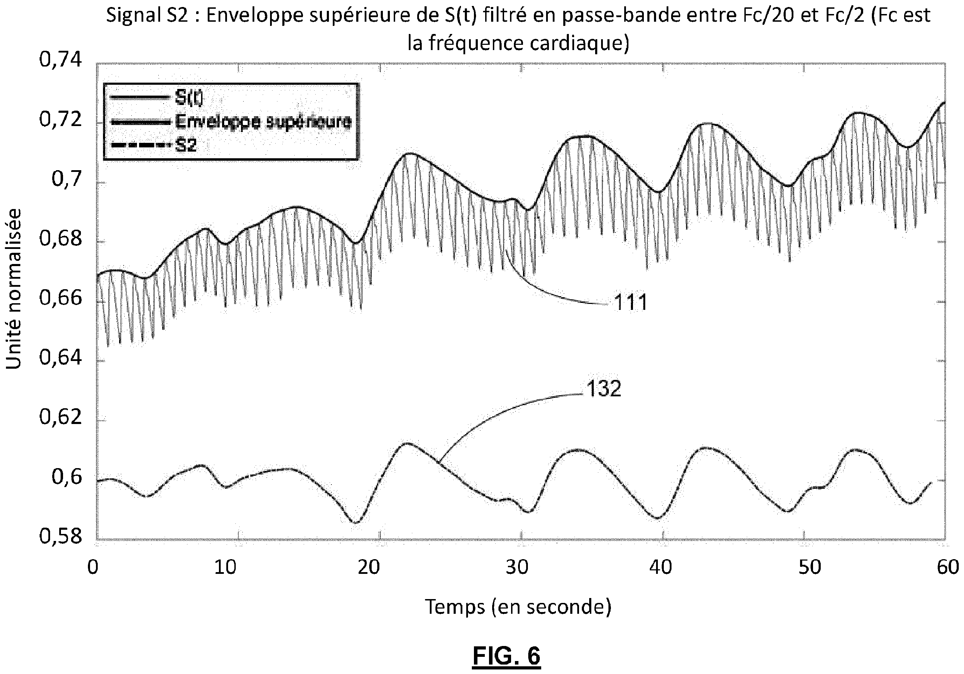

Le signal S2 132 est illustré en figure 6. Il est obtenu par :
a. détermination des sommets des battements de l'enveloppe supérieure de S'f(t),
b. puis par filtrage du signal des sommets des battements de l'enveloppe supérieure par un filtre passe-bande entre Fc/20 et Fc/2.

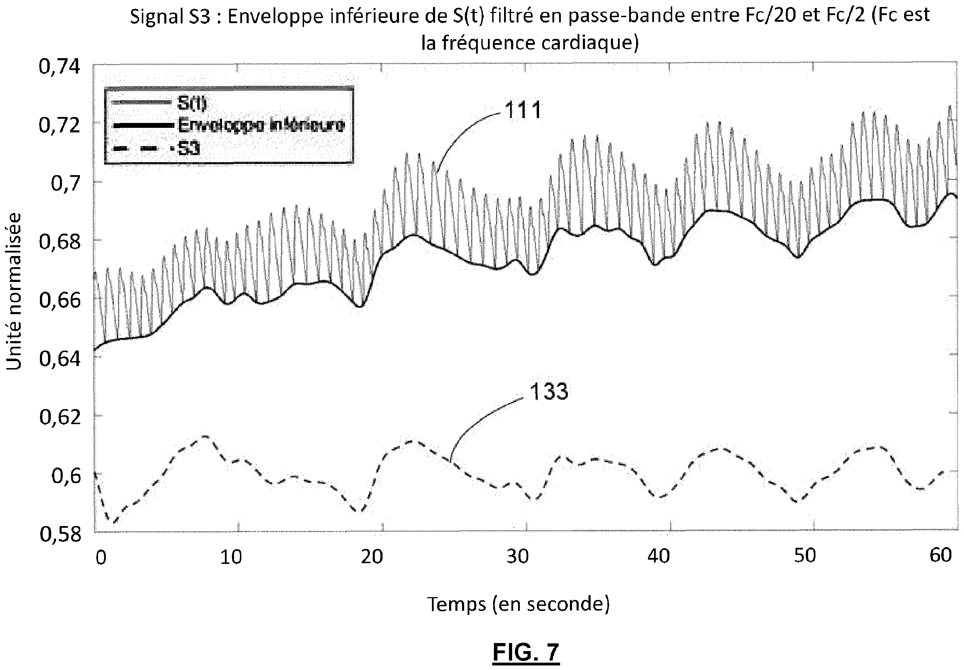

Le signal S3 133 est illustré en figure 7. Il est obtenu par :
a. détermination des vallées des battements de l'enveloppe inférieure de S'f(t),
b. puis par filtrage du signal des vallées des battements de l'enveloppe inférieure par un filtre passe-bande entre Fc/20 et Fc/2.

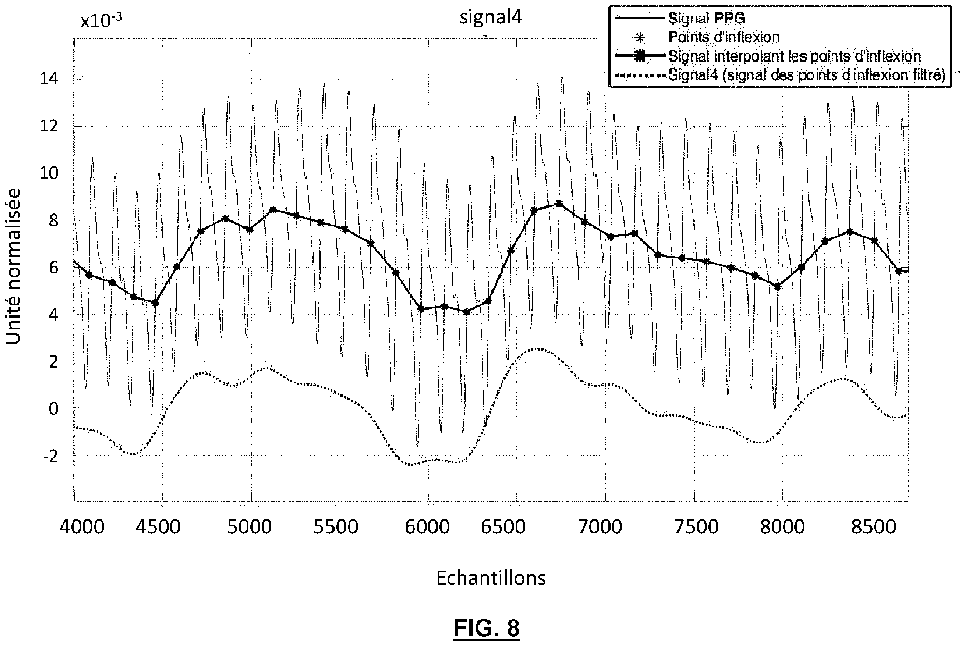

Le signal S4 134 est illustré en figure 8 ainsi que le signal PPG original S(t), S4 est alors obtenu par :
a. Génération d'une onde de vélocité du pouls VPG en effectuant une dérivée de première ordre du photoplethysmogramme S(t) par rapport à la coordonnée temporelle dudit photoplethysmogramme S(t) ;
b. Détermination des positions Pi des sommets de l'onde de vélocité du pouls VPG ;
c. Obtention d'un signal de variation des points d'inflexion en reportant des valeurs dudit photoplethysmogramme S(t) aux positions Pi ;
d. Sur-échantillonnage du signal de variation des points d'inflexion jusqu'à une fréquence Fs prédéterminée, de préférence généralement équivalente à celle du signal S(t);
e. Filtrage en passe-bande entre les fréquences Fc/20 et Fc/2 du signal suréchantillonné.

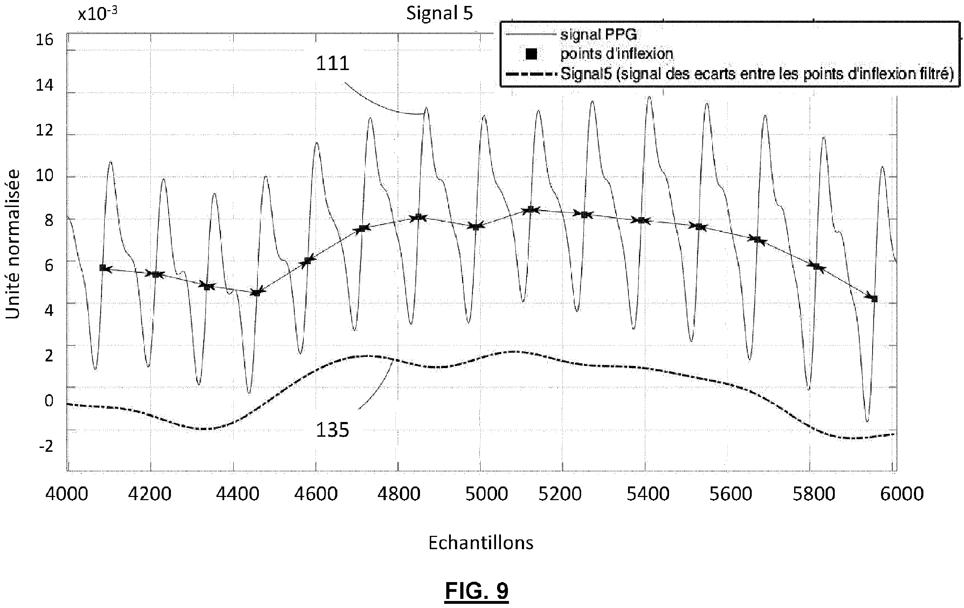

Le signal S5 135 est illustré en figure 9 ainsi que le signal PPG original S(t), S5 est alors obtenu par:
a. Localisation des points d'inflexion Pi dans une montée systolique d'un battement de coeur de l'utilisateur en identifiant la position des sommets de la dérivée de premier ordre de S(t) par rapport à la coordonnée temporelle de S(t)
b. Obtention d'un signal de variation des points d'inflexion Pi en reportant des valeurs de différences temporelles entre Pi et Pi-1;
c. Sur-échantillonnage de ce signal d'intervalles des points d'inflexion Pi jusqu'à une fréquence Fs prédéterminée, de préférence généralement du même ordre que S(t);
d. Filtrage en passe-bande entre les fréquences Fc/20 et Fc/2 du signal suréchantillonné ;

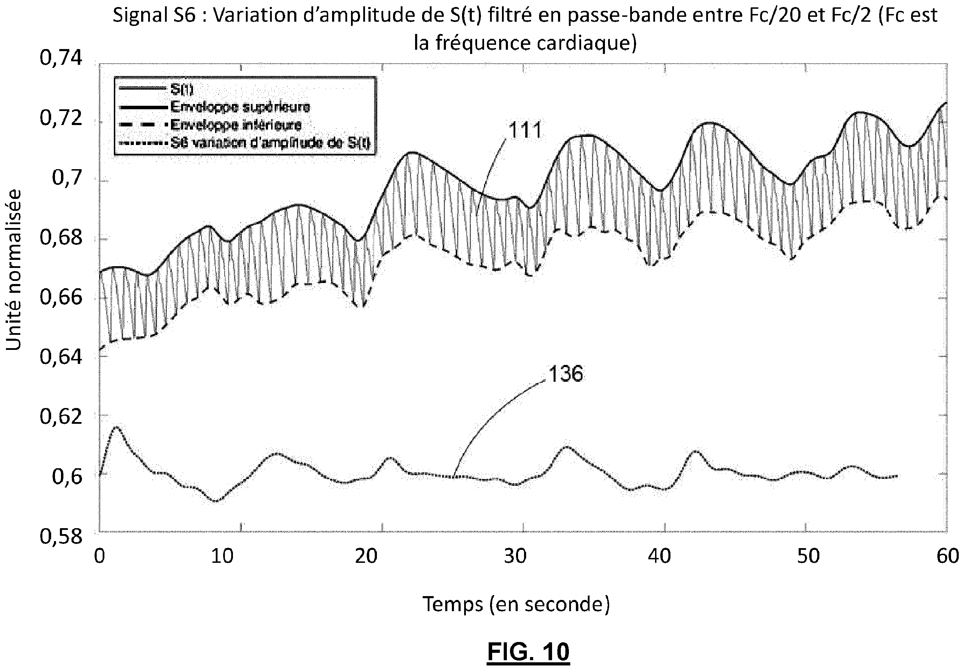

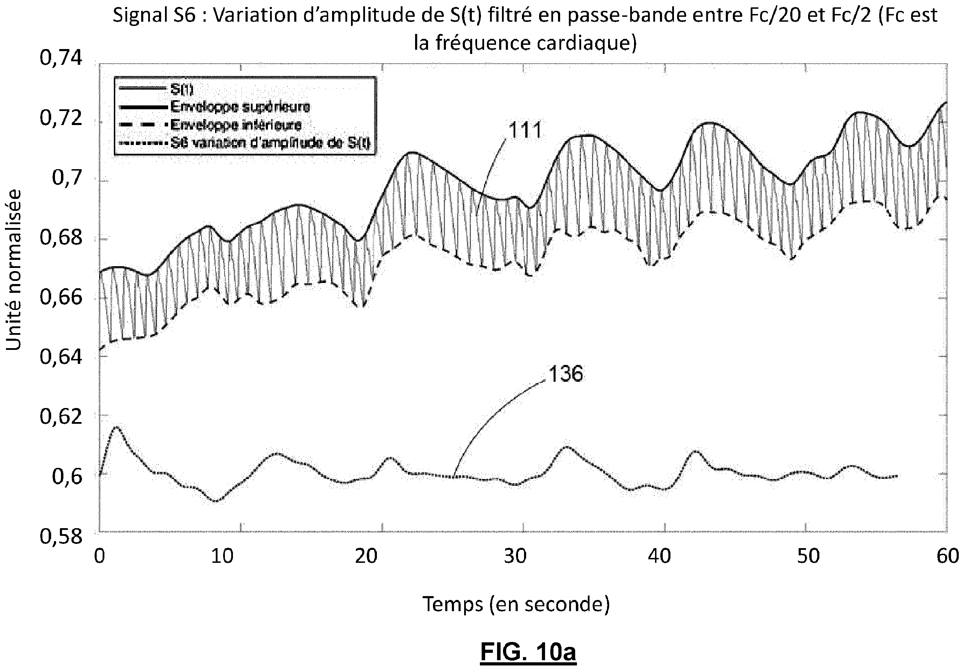

Le signal S6 136 est illustré en figure 10. Il obtenu par :
a. détermination des variations d'amplitude de S'f(t),
b. puis par filtrage par un filtre passe-bande entre Fc/20 et Fc/2 du signal de variations d'amplitude.

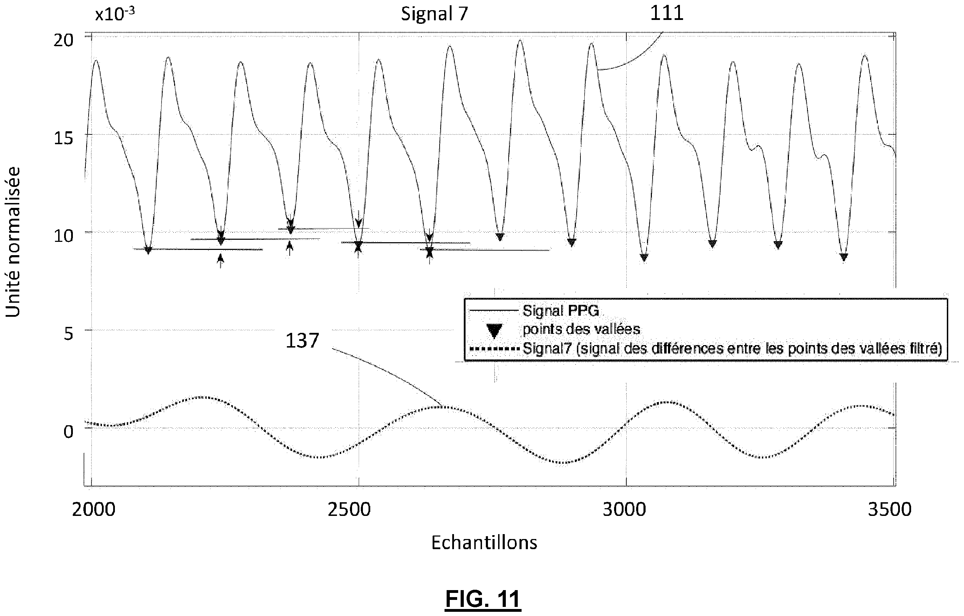

Le signal S7 137 est illustré en figure 11 ainsi que le signal PPG original S(t), S4 est alors obtenu par obtenu par :
a. détermination des vallées des battements de l'enveloppe inférieure du deuxième photoplethysmogramme filtré,
b. suréchantillonnage du signal des vallées des battements de l'enveloppe inférieure jusqu'à une fréquence Fs prédéterminée, de préférence généralement du même ordre que la fréquence du signal S(t) ;
c. Dérivation au premier ordre selon la coordonnée temporelle du signal suréchantillonné ;
d. puis par filtrage du signal dérivé par un filtre passe-bande entre Fc/20 et Fc/2.

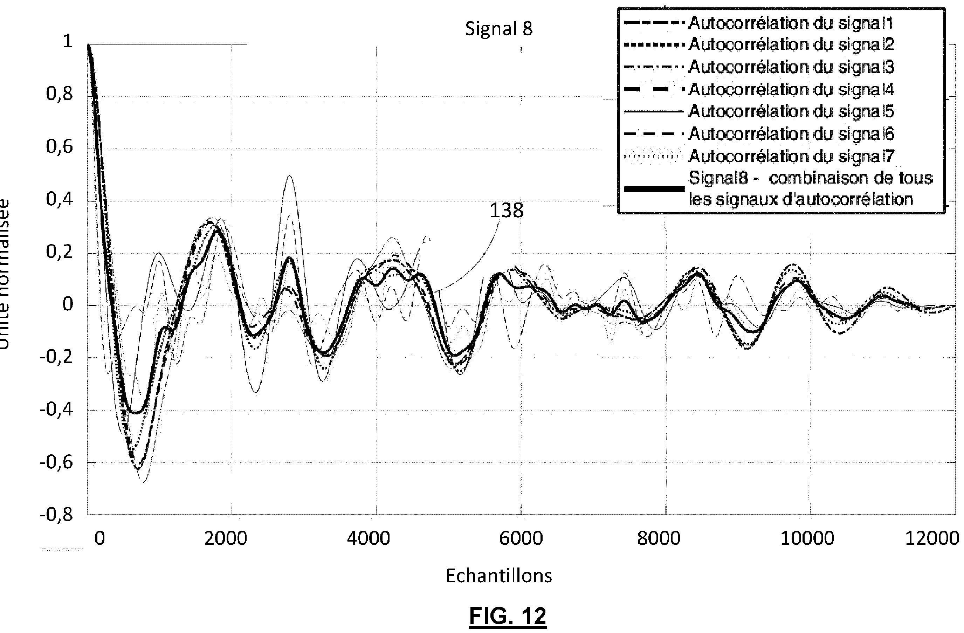

Le signal S8 138 est illustré en figure 12 ainsi que les signaux d'autocorrélation de S1, S2, S3, S4, S5, S6 et S7. Le signal S8 est généré à partir d'au moins 3, de préférence 4, avantageusement 5, de préférence 6 et avantageusement 7 signaux S1, S2, S3, S4, S5, S6 et S7. S8 et de préférence généré par les étapes suivantes :
a. Calcul respectivement pour chaque signal Sx de son signal d'autocorrélation normalisé Sxc ;
b. Le signal S8 est égal à la moyenne arithmétique des signaux d'autocorrélations normalisés Sxc.

De manière particulièrement avantageuse, certains desdits signaux S1 à S8 ont une tendance à mieux représenter une respiration lente et profonde (comme S1, S2, S3 par exemple) et d'autres représentent mieux une respiration rapide et peu profonde (comme S5, S6 et S7 par exemple). Le fait de combiner les signaux apporte une harmonisation de ces effets. De plus cette combinaison de signaux pour obtenir S8 utilise la fonction d'autocorrélation permettant de mettre en valeur les ondulations naturelles de la respiration présentes dans le signal cardiaque.

Selon un mode de réalisation préféré, l'ensemble des calculs et traitement des signaux de la présente invention est réalisé par l'unité de traitement de données.

Nous allons à présent décrire les algorithmes A1, A2, A3 et A4 précédemment discutés.

Avantageusement, ces algorithmes sont configurés pour être appliqués à chaque signal S1 à S8. Pour des questions de simplification, dans les paragraphes suivants, Sx désigne tout signal pris parmi au moins S1 à S8.

Selon un mode de réalisation, l'algorithme A1 141 comprend au moins les étapes suivantes appliquées au signal Sx :
a. Collecte des points de mesure correspondant à un croisement des abscisses par le signal considéré, soit Sx(t) = 0 ;
b. Calcul de l'intervalle moyen Tm entre chacun des points de traversé des abscisses ;
c. Calcul du taux respiratoire estimé Rx.A1 avec Rx.A1 = 60/(2*Tm) ;

A titre d'exemple non limitatif, l'algorithme A1 141 appliqué au signal S1 produit un taux respiratoire estimé R1.A1, pour le signal S2 cela sera R2.A1, et ainsi de suite pour les autres signaux.

Selon un mode de réalisation, l'algorithme A2 142 comprend au moins les étapes suivantes appliquées au signal Sx :
a. Collecte des points de mesure correspondant au sommet des ondulations du signal considéré ;
b. Calcul de l'intervalle moyen Ts entre chacun des points des sommets ;
c. Calcul du taux respiratoire estimé Rx.A2 avec Rx.A2 = 60/(Ts).

A titre d'exemple non limitatif, l'algorithme A2 142 appliqué au signal S4 produit un taux respiratoire estimé R4.A2, pour le signal S3 cela sera R3.A2, et ainsi de suite pour les autres signaux.

Selon un mode de réalisation, l'algorithme A3 143 comprend au moins les étapes suivantes appliquées au signal Sx :
a. Détermination de la densité spectrale de correntropie en calculant la transformée de Fourrier de l'autocorrentropie centrée du signal considéré ;
b. Extraction du taux respiratoire estimé Rx.A3 en déterminant le sommet de la densité spectrale de correntropie sur une plage fréquentielle prédéterminée, de préférence entre max(Fc/20, 0.05) et min(Fc/2,1) où 0.05Hz et 1 Hz correspondent respectivement à des taux respiratoires de 3 et 60 respirations par minute

A titre d'exemple non limitatif, l'algorithme A3 143 appliqué au signal S7 produit un taux respiratoire estimé R7.A3, pour le signal S8 cela sera R8.A3, et ainsi de suite pour les autres signaux.

Selon un mode de réalisation, l'algorithme A4 144 comprend au moins les étapes suivantes appliquées au signal Sx :
a. Calcul du signal d'autocorrélation du signal considéré ;
b. Collecte des points de mesure correspondant au sommet des ondulations du signal d'autocorrélation du signal considéré, de préférence correspondant au sommet des ondulations de la deuxième moitié du signal d'autocorrélation du signal considéré ;
c. Calcul de l'intervalle moyen Tas entre chacun des points des sommets ;
d. Calcul du taux respiratoire estimé Rx.A4 avec Rx.A4 = 60/(Tas).

A titre d'exemple non limitatif, l'algorithme A4 144 appliqué au signal S5 produit un taux respiratoire estimé R5.A4, pour le signal S6 cela sera R6.A4, et ainsi de suite pour les autres signaux.

De manière particulièrement avantageuse, ces algorithmes sont très complémentaires en termes de performance : si le signal respiratoire est de très bonne qualité, les quatre algorithmes convergeront vers le même résultat. Par contre, si le signal respiratoire présente des artéfacts, les algorithmes A1, A2, A3 et A4 vont avoir des sensibilités différentes exploitables pour minimiser l'erreur d'estimation du taux respiratoire final.

Selon un mode de réalisation préféré, l'ensemble des algorithmes A1, A2, A3 et A4 sont mis en oeuvre par l'unité de traitement de données. Selon un mode de réalisation une partie au moins de ces algorithmes peut être mise en oeuvre via un serveur informatique.

Nous allons à présent décrire le calcul des taux respiratoire r1, r2, r3 et r4 à partir d'une partie au moins des taux respiratoires estimés précédemment Rx.Ay.

L'avantage de l'utilisation du calcul de la médiane sur des sous-ensembles de taux respiratoires estimés est de minimiser le taux d'erreur si la moitié de ces taux divergent de la vrai valeur. Contrairement à la moyenne qui va garder proportionnellement l'effet de cette divergence dans le résultat final.

Selon un mode de réalisation, le premier taux respiratoire intermédiaire r1 151 est égal à la médiane des médianes des taux respiratoires estimés Rx.Ay d'un signal Sx, Sx étant pris parmi au moins S1 à S7.

Ainsi, dans le cas où tous les signaux S1 à S7 sont considérés, r1 = médiane( médiane ((R1.A1 , R1.A2 , R1.A3 , R1.A4)) , médiane((R2.A1 , R2.A2 , R2.A3 , R2.A4)) , médiane ((R3.A1 , R3.A2 , R3.A3 , R3.A4)) , médiane ((R4.A1 , R4.A2 , R4.A3 , R4.A4)) , médiane ((R5.A1 , R5.A2 , R5.A3 , R5.A4)) , médiane ((R6.A1 , R6.A2 , R6.A3 , R6.A4)) , médiane ((R7.A1 , R7.A2 , R7.A3 , R7.A4))).

Selon un mode de réalisation, le deuxième taux respiratoire intermédiaire r2 152 correspondant au sommet de l'histogramme formé par les taux respiratoires estimés Rx.Ay de chaque signal Sx considéré pris parmi S1 à S7 traité au travers de chaque algorithme Ay considéré pris parmi A1 à A4.

Ainsi par exemple, en considérant l'ensemble des signaux S1 à S7 et l'ensemble des algorithmes A1 à A4, r2 correspond au sommet de l'histogramme formé par les valeurs suivantes : R1.A1, R1.A2, R1.A3, R1.A4, R2.A1, R2.A2, R2.A3, R2.A4, R3.A1, R3.A2, R3.A3, R3.A4, R4.A1, R4.A2, R4.A3, R4.A4, R5.A1, R5.A2, R5.A3, R5.A4, R6.A1, R6.A2, R6.A3, R6.A4, R7.A1, R7.A2, R7.A3, R7.A4.

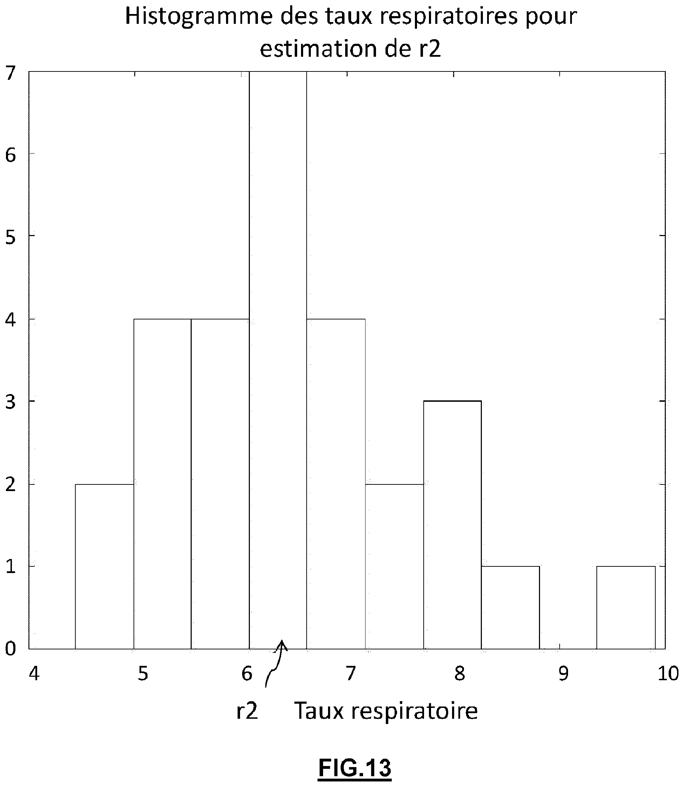

La figure 13 illustre un tel histogramme selon un exemple non limitatif.

Selon un mode de réalisation, le troisième taux respiratoire intermédiaire r3 153 est égal à la valeur médiane des taux respiratoires estimés pour chaque signal considéré traité par le troisième algorithme A3.

Ainsi par exemple dans le cas où tous les signaux S1 à S7 sont considérés, r3 = médiane(R1.A3 , R2.A3, R3.A3 , R4.A3 , R5.A3 , R6.A3 , R7.A3).

Selon un mode de réalisation, le quatrième taux respiratoire intermédiaire r4 154 est égal à la valeur médiane des taux respiratoires estimés du signal S8.

Ainsi par exemple dans le cas où l'ensemble des algorithmes A1 à A4 traite le signal S8, r4 = médiane(R8.A1 , R8.A2 , R8.A3 , R8.A4).

Ensuite comme indiqué précédemment, le taux respiratoire R de l'utilisateur est calculé en réalisant la médiane d'une partie au moins des taux respiratoires intermédiaires calculés précédemment. Ici encore, l'ensemble des calculs mis en oeuvre par le procédé de la présente invention est de préférence exécuté par l'unité de traitement de données.

La présente invention permet ainsi de déterminer le taux respiratoire d'un utilisateur de manière précise et fiable en mesurant simplement son rythme cardiaque et en particulier un signal photoplethysmogramme S(t).

Les traitements et algorithmiques appliqués alors à ce signal permettent de réduire les bruit des mesures et d'exploiter une mesure simple et peu coûteuse pour déterminer avec précision et fiabilité le taux respiratoire d'un utilisateur, et cela en un temps très court.

L'invention n'est pas limitée aux modes de réalisations précédemment décrits et s'étend à tous les modes de réalisation couverts par les revendications.

### REFERENCES

- 1: Doigt de l'utilisateur
- 10: Dispositif de mesure
- 11: Source lumineuse
- 11a: Rayons lumineux transmis
- 11b: Rayons lumineux réfléchis
- 12: Capteur optique
- 100: Procédé de détermination du taux respiratoire R
- 110: Capture du signal photoplethysmogramme S(t)
- 111: Signal photoplethysmogramme S(t)
- 120: Filtrage du photoplethysmogramme S(t) et estimation de la fréquence cardiaque Fc
- 130: Obtention de 8 signaux
- 131: Signal S1
- 132: Signal S2
- 133: Signal S3
- 134: Signal S4
- 135: Signal S5
- 136: Signal S6
- 137: Signal S7
- 138: Signal S8
- 140: Traitement algorithmique des signaux
- 141: Premier algorithme
- 142: Deuxième algorithme
- 143: Troisième algorithme
- 144: Quatrième algorithme
- 150: Calcul des taux respiratoires intermédiaires
- 151: Premier taux respiratoire intermédiaire r1
- 152: Deuxième taux respiratoire intermédiaire r2
- 153: Troisième taux respiratoire intermédiaire r3
- 154: Quatrième taux respiratoire intermédiaire r4
- 160: Calcul du taux respiratoire R

## Revendications

1. Procédé (100) de détermination du taux respiratoire R d'au moins un utilisateur comprenant au moins les étapes suivantes mises en oeuvre par au moins une unité de traitement de données comprenant :
a. Acquisition d'au moins un photoplethysmogramme S(t) dudit utilisateur, l'étape d'acquisition dudit photoplethysmogramme S(t) comprenant au moins l'une parmi les étapes suivantes :
∘ Mesure, de préférence à une fréquence supérieure ou égale à 50Hz, dudit au moins un photoplethysmogramme S(t) par un dispositif de mesure, de préférence porté ou destiné à être porté par ledit au moins un utilisateur, le dispositif comprenant au moins un capteur (12) apte à mesurer au moins un photoplethysmogramme S(t) ;
∘ Réception depuis au moins une base de données dudit au moins un photoplethysmogramme S(t) ;
b. Estimation (120) de la fréquence cardiaque Fc à partir dudit au moins un photoplethysmogramme S(t) acquis dudit au moins un utilisateur ;
c. Obtention (130) d'au moins trois signaux (S1, ...S7) distincts les uns des autres, chaque signal obtenu (S1, ...S7) étant fonction dudit au moins un photoplethysmogramme S(t) acquis ;
d. Génération (138) d'un signal S8 à partir desdits au moins trois signaux obtenus (S1, ..., S7) ;
e. Traitement algorithmique (140) de chacun des au moins trois signaux obtenus (S1, ..., S7) et du signal S8 par au moins deux algorithmes Ai et Aj, de sorte à obtenir au moins deux taux respiratoires estimés, respectivement Rx.Ai et Rx.Aj, pour chaque signal Sx traité respectivement par l'algorithme Ai et par l'algorithme Aj, chaque signal Sx étant pris parmi lesdits au moins trois signaux obtenus (S1, ..., S7) et le signal S8; les au moins deux algorithmes Ai et Aj étant distincts l'un de l'autre ;
f. Calcul (150) d'au moins deux taux respiratoires intermédiaires rm et rn à partir des taux respiratoires estimés (Rx.Ai, Rx.Aj) ;
g. Détermination (160) du taux respiratoire R en calculant la médiane desdits au moins deux taux respiratoires intermédiaires rm et rn.

2. Procédé (100) selon la revendication précédente comprenant, avant l'étape d'estimation de la fréquence cardiaque Fc, une étape de filtrage dudit photoplethysmogramme, de préférence par un filtre passe-bande, de sorte à générer un premier photoplethysmogramme filtré.

3. Procédé (100) selon la revendication précédente comprenant, après l'étape d'estimation de la fréquence cardiaque Fc, une étape additionnelle de filtrage dudit premier photoplethysmogramme filtré entre Fc/20 et 4Hz de sorte à générer un deuxième photoplethysmogramme filtré.

4. Procédé (100) selon la revendication précédente dans lequel lesdits au moins trois signaux (S1, ..., S7) distincts obtenus sont pris parmi au moins les signaux suivants S1, S2, S3, S4, S5, S6 et S7 :
a. un signal S1 (131) obtenu par :
∘ Application d'un filtre passe-bande entre Fc/20 et Fc/2 au deuxième photoplethysmogramme filtré ;
b. un signal S2 (132) obtenu par :
∘ détermination des sommets des battements de l'enveloppe supérieure du deuxième photoplethysmogramme filtré ;
∘ puis par filtrage du signal des sommets des battements de l'enveloppe supérieure par un filtre passe-bande entre Fc/20 et Fc/2.
c. un signal S3 (133) obtenu par :
∘ détermination des vallées des battements de l'enveloppe inférieure du deuxième photoplethysmogramme filtré ;
∘ puis par filtrage du signal des vallées des battements de l'enveloppe inférieure par un filtre passe-bande entre Fc/20 et Fc/2 ;
d. un signal S4 (134) obtenu par :
∘ Génération d'une onde de vélocité du pouls VPG en effectuant une dérivée de première ordre du photoplethysmogramme S(t) par rapport à la coordonnée temporelle dudit photoplethysmogramme S(t) ;
∘ Détermination des positions Pi des sommets de l'onde de vélocité du pouls VPG ;
∘ Obtention d'un signal de variation des points d'inflexion en reportant des valeurs dudit photoplethysmogramme S(t) aux positions Pi ;
∘ Sur-échantillonnage du signal de variation des points d'inflexion jusqu'à une fréquence Fs prédéterminée ;
∘ Filtrage en passe-bande entre les fréquences Fc/20 et Fc/2 du signal suréchantillonné ;
e. un signal S5 (135) obtenu par :
∘ Localisation des points d'inflexion Pi dans une montée systolique d'un battement de coeur de l'utilisateur en identifiant la position des sommets de la dérivée de premier ordre du photoplethysmogramme S(t) par rapport à la coordonnée temporelle dudit photoplethysmogramme S(t) ;
∘ Obtention d'un signal de variation des points d'inflexion Pi en reportant des valeurs de différences temporelles entre Pi et Pi-1 ;
∘ Sur-échantillonnage de ce signal d'intervalles des points d'inflexion Pi jusqu'à une fréquence Fs prédéterminée ;
∘ Filtrage en passe-bande entre les fréquences Fc/20 et Fc/2 du signal suréchantillonné ;
f. un signal S6 (136) obtenu par :
∘ détermination des variations d'amplitude du deuxième photoplethysmogramme filtré ;
∘ puis par filtrage par un filtre passe-bande entre Fc/20 et Fc/2 du signal de variations d'amplitude ;
g. un signal S7 (137) obtenu par :
∘ détermination des vallées des battements de l'enveloppe inférieure du deuxième photoplethysmogramme filtré ;
∘ suréchantillonnage du signal des vallées des battements de l'enveloppe inférieure jusqu'à une fréquence Fs prédéterminée ;
∘ Dérivation au premier ordre selon la coordonnée temporelle du signal suréchantillonné ;
∘ puis par filtrage du signal dérivé par un filtre passe-bande entre Fc/20 et Fc/2.

5. Procédé (100) selon l'une quelconque des revendications précédentes dans lequel l'étape de génération d'un signal S8 (138) à partir desdits au moins trois signaux obtenus (S1, ...S7) comprend au moins les étapes suivantes :
a. Calcul, pour chaque signal parmi lesdits au moins trois signaux obtenus (S1, ..., S7), d'un signal d'autocorrélation normalisé ;
b. Calcul de la moyenne arithmétique des signaux d'autocorrélations normalisés.

6. Procédé (100) selon l'une quelconque des revendications précédentes dans lequel les au moins deux algorithmes Ai et Aj distincts l'un de l'autre sont pris parmi au moins les algorithmes A1, A2, A3 et A4 suivants :
a. un algorithme A1 (141) comprenant au moins les étapes suivantes appliquées au signal Sx:
∘ Collecte des points de mesure correspondant à un croisement des abscisses par le signal considéré, soit Sx(t) = 0 ;
∘ Calcul de l'intervalle moyen Tm entre chacun des points de traversé des abscisses ;
∘ Calcul de Rx.A1 avec Rx.A1 = 60/(2*Tm) ;
b. un algorithme A2 (142) comprenant au moins les étapes suivantes appliquées au signal Sx:
∘ Collecte des points de mesure correspondant au sommet des ondulations du signal considéré ;
∘ Calcul de l'intervalle moyen Ts entre chacun des points des sommets ;
∘ Calcul de Rx.A2 avec Rx.A2 = 60/(Ts) ;
c. un algorithme A3 (143) comprenant au moins les étapes suivantes appliquées au signal Sx:
∘ Détermination de la densité spectrale de correntropie en calculant la transformée de Fourrier de l'autocorrentropie centrée du signal considéré ;
∘ Extraction de Rx.A3 en déterminant le sommet de la densité spectrale de correntropie sur une plage fréquentielle prédéterminée ;
d. un algorithme A4 (144) comprenant au moins les étapes suivantes appliquées au signal Sx:
∘ Calcul du signal d'autocorrélation du signal considéré ;
∘ Collecte des points de mesure correspondant au sommet des ondulations du signal d'autocorrélation du signal considéré ;
∘ Calcul de l'intervalle moyen Tas entre chacun des points des sommets ;
∘ Calcul de Rx.A4 avec Rx.A4 = 60/(Tas).

7. Procédé (100) selon la revendication précédente dans lequel les au moins deux taux respiratoires intermédiaires rm et rn sont pris parmi au moins les taux respiratoires intermédiaires r1, r2, r3 et r4 suivants :
a. r1 (151) est égal à la valeur médiane des taux respiratoires estimés Rx.Ay d'un signal Sx, Sx étant pris parmi au moins les signaux obtenus ;
b. r2 (152) correspond au sommet de l'histogramme formé par les taux respiratoires estimés Rx.Ay de chaque signal Sx considéré pris parmi les signaux obtenus traités au travers de chaque algorithme Ay considéré pris parmi A1 à A4 ;
c. r3 (153) est égal à la valeur médiane des taux respiratoires estimés Rx.A3 pour chaque signal Sx considéré pris parmi les signaux obtenus traité par l'algorithme A3 ;
d. r4 (154) est égal à la valeur médiane des taux respiratoires estimés R8.Ay du signal S8 traité au travers de chaque algorithme Ay considéré pris parmi A1 à A4.

8. Procédé (100) selon l'une quelconque des revendications précédentes dans lequel l'étape d'obtention d'au moins trois signaux (S1, ..., S7) distincts les uns des autres comprend l'obtention d'au moins 4, de préférence d'au moins 5, avantageusement d'au moins 6 et avantageusement des 7 signaux distincts S1, S2, S3, S4, S5, S6 et S7.

9. Procédé (100) selon l'une quelconque des revendications précédentes en combinaison avec la revendication 6 dans lequel l'étape de traitement algorithmique comprend l'utilisation d'au moins 3 et de préférence des 4 algorithmes distincts A1, A2, A3 et A4 appliqué à chacun des signaux obtenus (S1, ..., S7) et du signal S8.

10. Procédé (100) selon les deux revendications précédentes prises en combinaison dans lequel r1 (151) = médiane( médiane ((R1.A1, R1.A2, R1.A3, R1.A4)), médiane((R2.A1, R2.A2, R2.A3, R2.A4)) , médiane ((R3.A1, R3.A2, R3.A3, R3.A4)) , médiane ((R4.A1, R4.A2, R4.A3, R4.A4)) , médiane ((R5.A1, R5.A2, R5.A3, R5.A4)) , médiane ((R6.A1, R6.A2, R6.A3, R6.A4)) , médiane ((R7.A1, R7.A2, R7.A3, R7.A4))).

11. Procédé (100) selon les revendications 7, 8 et 9 prises en combinaison :
- dans lequel r2 (152) correspond au sommet de l'histogramme formé par les valeurs suivantes : R1.A1, R1.A2, R1.A3, R1.A4, R2.A1, R2.A2, R2.A3, R2.A4, R3.A1, R3.A2, R3.A3, R3.A4, R4.A1, R4.A2, R4.A3, R4.A4, R5.A1, R5.A2, R5.A3, R5.A4, R6.A1, R6.A2, R6.A3, R6.A4, R7.A1, R7.A2, R7.A3, R7.A4
et/ou
- dans lequel r3 (153) = médiane (R1.A3, R2.A3, R3.A3, R4.A3, R5.A3, R6.A3, R7.A3).

12. Procédé (100) selon l'une quelconque des revendications précédentes prises en combinaison avec la revendication 8 dans lequel S8 (138) = (S1 c+S2c+S3c+S4c+S5c+S6c+S7c)/7, avec les signaux Sxc correspondant au signal d'autocorrélation normalisé du signal Sx pris parmi S1, S2, S3, S4, S5, S6 et S7.

13. Produit programme d'ordinateur, de préférence enregistré sur un support non transitoire, comprenant des instructions, qui lorsqu'elles sont effectuées par au moins l'un parmi un processeur et un ordinateur, font que l'au moins un parmi le processeur et l'ordinateur, exécute le procédé (100) selon l'une quelconque des revendications précédentes.

14. Dispositif de mesure (10) d'un photoplethysmogramme S(t) (111) d'un utilisateur connecté à au moins une unité de traitement de données comprenant au moins une mémoire non transitoire comprenant un produit programme d'ordinateur selon la revendication précédente, le dispositif de mesure (10) comprenant au moins un capteur (12) apte à mesurer un photoplethysmogramme.

15. Dispositif (10) selon la revendication précédente dans lequel le capteur apte à mesurer un photoplethysmogramme est un capteur pris parmi au moins : capteur optique (12), capteur électrique, capteur radar, capteur de force, capteur de pression, capteur de vibration, capteur audio, capteur séismique.

## Patentansprüche

1. Verfahren (100) zum Bestimmen der Atemfrequenz R mindestens eines Benutzers, das mindestens die folgenden Schritte umfasst, die von mindestens einer Datenverarbeitungseinheit durchgeführt werden, umfassend:
a. Erfassen mindestens eines Photoplethysmogramms S(t) des Benutzers, wobei der Schritt des Erfassens des Photoplethysmogramms S(t) mindestens einen der folgenden Schritte umfasst:
o Messen, vorzugsweise bei einer Frequenz größer oder gleich 50 Hz, des mindestens einen Photoplethysmogramms S(t) durch eine Messvorrichtung, die vorzugsweise von dem mindestens einen Benutzer getragen wird oder vorgesehen ist, von diesem getragen zu werden, wobei die Vorrichtung mindestens einen Sensor (12) umfasst, der dazu ausgelegt ist, mindestens ein Photoplethysmogramm S(t) zu messen;
o Empfangen des mindestens einen Photoplethysmogramms S(t) von mindestens einer Datenbank;
b. Schätzen (120) der Herzfrequenz Fc anhand des mindestens einen Photoplethysmogramms S(t), das von dem mindestens einen Benutzer erfasst wurde;
c. Erhalten (130) von mindestens drei voneinander verschiedenen Signalen (S1, ..., S7), wobei jedes erhaltene Signal (S1, ..., S7) die Funktion des mindestens einen erfassten Photoplethysmogramms S(t) ist;
d. Erzeugen (138) eines Signals S8 anhand der mindestens drei erhaltenen Signale (S1, ..., S7);
e. algorithmisches Verarbeiten (140) jedes der mindestens drei erhaltenen Signale (S1, ..., S7) und des Signals S8 durch mindestens zwei Algorithmen Ai und Aj, um mindestens zwei geschätzte Atemfrequenzen, Rx.Ai bzw. Rx.Aj, für jedes vom Algorithmus Ai bzw. vom Algorithmus Aj verarbeitete Signal Sx zu erhalten, wobei jedes Signal Sx aus den mindestens drei erhaltenen Signalen (S1, ..., S7) und dem Signal S8 entnommen wird; wobei die mindestens zwei Algorithmen Ai und Aj voneinander verschieden sind;
f. Berechnen (150) von mindestens zwei Zwischenatemfrequenzen rm und rn anhand der geschätzten Atemfrequenzen (Rx.Ai, Rx.Aj);
g. Bestimmen (160) der Atemfrequenz R, indem der Mittelwert der mindestens zwei Zwischenatemfrequenzen rm und rn berechnet wird.

2. Verfahren (100) nach dem vorhergehenden Anspruch, das vor dem Schritt des Schätzens der Herzfrequenz Fc einen Schritt des Filterns des Photoplethysmogramms umfasst, vorzugsweise durch ein Bandpassfilter, um ein erstes gefiltertes Photoplethysmogramm zu erzeugen.

3. Verfahren (100) nach dem vorhergehenden Anspruch, das nach dem Schritt des Schätzens der Herzfrequenz Fc einen zusätzlichen Schritt des Filterns des ersten gefilterten Photoplethysmogramms zwischen Fc/20 und 4 Hz umfasst, um ein zweites gefiltertes Photoplethysmogramm zu erzeugen.

4. Verfahren (100) nach dem vorhergehenden Anspruch, wobei die mindestens drei voneinander verschiedenen erhaltenen Signale (S1, ..., S7) aus mindestens den folgenden Signalen S1, S2, S3, S4, S5, S6 und S7 entnommen wurden:
a. ein Signal S1 (131), erhalten durch:
o Anwenden eines Bandpassfilters zwischen Fc/20 und Fc/2 auf das zweite gefilterte Photoplethysmogramm;
b. ein Signal S2 (132), erhalten durch:
o Bestimmen der Spitzen der Schläge der oberen Hüllkurve des zweiten gefilterten Photoplethysmogramms;
o anschließendes Filtern des Signals der Spitzen der Schläge der oberen Hüllkurve durch ein Bandpassfilter zwischen Fc/20 und Fc/2;
c. ein Signal S3 (133), erhalten durch:
o Bestimmen der Täler der Schläge der unteren Hüllkurve des zweiten gefilterten Photoplethysmogramms;
o anschließendes Filtern des Signals der Täler der Schläge der unteren Hüllkurve durch ein Bandpassfilter zwischen Fc/20 und Fc/2;
d. ein Signal S4 (134), erhalten durch:
o Erzeugen einer Pulsgeschwindigkeitswelle VPG, indem eine Ableitung erster Ordnung des Photoplethysmogramms S(t) in Bezug auf die zeitliche Koordinate des Photoplethysmogramms S(t) ausgeführt wird;
o Bestimmen der Positionen Pi der Spitzen der Pulsgeschwindigkeitswelle VPG;
o Erhalten eines Änderungssignals der Wendepunkte, indem Werte des Photoplethysmogramms S(t) auf Positionen Pi übertragen werden;
o Überabtasten des Änderungssignals der Übergangspunkte bis zu einer vorbestimmten Frequenz Fs;
o Bandpassfiltern zwischen den Frequenzen Fc/20 und Fc/2 des überabgetasteten Signals;
e. ein Signal S5 (135), erhalten durch:
o Lokalisieren der Übergangspunkte Pi in einem systolischen Anstieg eines Herzschlags des Benutzers, indem die Position der Spitzen der Ableitung erster Ordnung des Photoplethysmogramms S (t) in Bezug auf die zeitliche Koordinate des Photoplethysmogramms S(t) identifiziert wird;
o Erhalten eines Änderungssignals der Übergangspunkte Pi, indem Werte zeitlicher Differenzen zwischen Pi und Pi-1 übertragen werden;
o Überabtasten dieses Signals von Intervallen der Übergangspunkte Pi bis zu einer vorbestimmten Frequenz Fs;
o Bandpassfiltern zwischen den Frequenzen Fc/20 und Fc/2 des überabgetasteten Signals;
f. ein Signal S6 (136), erhalten durch:
o Bestimmen der Amplitudenänderungen des zweiten gefilterten Photoplethysmogramms;
o anschließendes Filtern des Amplitudenänderungssignals mit einem Bandpassfilter zwischen Fc/20 und Fc/2;
g. ein Signal S7 (137), erhalten durch:
o Bestimmen der Täler der Schläge der unteren Hüllkurve des zweiten gefilterten Photoplethysmogramms;
o Überabtasten des Signals der Täler der Schläge der unteren Hüllkurve bis zu einer vorbestimmten Frequenz Fs;
o Ableiten erster Ordnung entsprechend der zeitlichen Koordinate des überabgetasteten Signals;
o anschließendes Filtern des abgeleiteten Signals durch ein Bandpassfilter zwischen Fc/20 und Fc/2.

5. Verfahren (100) nach einem der vorhergehenden Ansprüche, wobei der Schritt des Erzeugens eines Signals S8 (138) aus den mindestens drei erhaltenen Signalen (S1, ..., S7) mindestens die folgenden Schritte umfasst:
a. Berechnen eines normalisierten Autokorrelationssignals für jedes Signal aus den mindestens drei erhaltenen Signalen (S1, ..., S7);
b. Berechnen des arithmetischen Mittels normalisierter Autokorrelationssignale.

6. Verfahren (100) nach einem der vorhergehenden Ansprüche, wobei die mindestens zwei voneinander verschiedenen Algorithmen Ai und Aj aus mindestens den folgenden Algorithmen A1, A2, A3 und A4 entnommen werden:
a. ein Algorithmus A1 (141), der mindestens die folgenden Schritte umfasst, die auf das Signal Sx angewendet werden:
o Sammeln von Messpunkten, die einem Schnittpunkt der Abszissen durch das betrachtete Signal entsprechen, d. h. Sx(t) = 0;
o Berechnen des durchschnittlichen Intervalls Tm zwischen jedem der Abszissenkreuzungspunkte;
o Berechnen von Rx.A1, wobei Rx.A1 = 60/(2*Tm);
b. einen Algorithmus A2 (142), der mindestens die folgenden Schritte umfasst, die auf das Signal Sx angewendet werden:
o Sammeln von Messpunkten, die der Spitze der Wellen des betrachteten Signals entsprechen;
o Berechnen des durchschnittlichen Intervalls Ts zwischen jedem der Spitzenpunkte;
o Berechnen von Rx.A2, wobei Rx.A2 = 60/(Ts);
c. ein Algorithmus A3 (143), der mindestens die folgenden Schritte umfasst, die auf das Signal Sx angewendet werden:
o Bestimmen der spektralen Dichte der Korrentropie, indem die Fourier-Transformation der zentrierten Autokorrentropie des betrachteten Signals berechnet wird;
o Extrahieren von Rx.A3, indem die Spitze der spektrale Korrentropiedichte über einen vorbestimmten Frequenzbereich bestimmt wird;
d. ein Algorithmus A4 (144), der mindestens die folgenden Schritte umfasst, die auf das Signal Sx angewendet werden:
o Berechnen des Autokorrelationssignals des betrachteten Signals;
o Sammeln von Messpunkten, die der Spitze der Wellen des Autokorrelationssignals des betrachteten Signals entsprechen;
o Berechnen des durchschnittlichen Intervalls Tas zwischen jeder der Spitzen;
o Berechnen von Rx.A4, wobei Rx.A4 = 60/(Tas).

7. Verfahren (100) nach dem vorhergehenden Anspruch, wobei die mindestens zwei Zwischenatemfrequenzen rm und rn aus mindestens den folgenden Zwischenatemfrequenzen r1, r2, r3 und r4 genommen werden:
a. r1 (151) ist gleich dem Mittelwert der geschätzten Atemfrequenzen Rx.Ay eines Signals Sx ist, wobei Sx zumindest aus den erhaltenen Signalen entnommen wird;
b. r2 (152) entspricht der Spitze des Histogramms, das durch die geschätzten Atemfrequenzen Rx.Ay jedes betrachteten Signals Sx gebildet wird, das aus den erhaltenen Signalen, die durch jeden betrachteten Algorithmus Ay verarbeitet wurden, von A1 bis A4 entnommen wird;
c. r3 (153) ist gleich dem Mittelwert der geschätzten Atemfrequenzen Rx.A3 für jedes betrachtete Signal Sx, das aus den vom Algorithmus A3 verarbeiteten Signalen entnommen wird;
d. r4 (154) ist gleich dem Mittelwert der geschätzten Atemfrequenzen R8.Ay des Signals S8, das durch jeden betrachteten Algorithmus Ay von A1 bis A4 verarbeitet wurde.

8. Verfahren (100) nach einem der vorhergehenden Ansprüche, wobei der Schritt des Erhaltens von mindestens drei voneinander verschiedenen Signalen (S1, ..., S7) das Erhalten von mindestens 4, vorzugsweise mindestens 5, vorteilhafterweise mindestens 6 und vorteilhafterweise 7 unterschiedlichen Signalen S1, S2, S3, S4, S5, S6 und S7 umfasst.

9. Verfahren (100) nach einem der vorhergehenden Ansprüche in Kombination mit Anspruch 6, wobei der Schritt des algorithmischen Verarbeitens das Verwenden von mindestens 3 und vorzugsweise 4 unterschiedlichen Algorithmen A1, A2, A3 und A4 umfasst, die auf jedes der erhaltenen Signale (S1, ..., S7) und auf das Signal S8 angewendet werden.

10. Verfahren (100) nach den beiden vorhergehenden Ansprüchen in Kombination, wobei r1 (151) = Mittelwert (Mittelwert((R1.A1, R1.A2, R1.A3, R1.A4)), Mittelwert ((R2.A1, R2.A2, R2.A3, R2.A4)), Mittelwert ((R3.A1, R3.A2, R3.A3, R3.A4)), Mittelwert ((R4.A1, R4.A2, R4.A3, R4.A4)), Mittelwert ((R5.A1, R5.A2, R5.A3, R5.A4)), Mittelwert ((R6.A1, R6.A2, R6.A3, R6.A4)), Mittelwert ((R7.A1, R7.A2, R7.A3, R7.A4))).

11. Verfahren (100) nach den Ansprüchen 7, 8 und 9 in Kombination:
- wobei r2 (152) der Spitze des Histogramms entspricht, das aus den folgenden Werten gebildet wird: R1.A1, R1.A2, R1.A3, R1.A4, R2.A1, R2.A2, R2.A3, R2.A4, R3.A1, R3.A2, R3.A3, R3.A4, R4.A1, R4.A2, R4.A3, R4.A4, R5.A1, R5.A2, R5.A3, R5.A4, R6.A1, R6.A2, R6.A3, R6.A4, R7.A1, R7.A2, R7.A3, R7.A4
und/oder
- wobei r3 (153) = Mittelwert (R1.A3, R2.A3, R3.A3, R4.A3, R5.A3, R6.A3, R7.A3).

12. Verfahren (100) nach einem der vorhergehenden Ansprüche in Kombination mit Anspruch 8, wobei S8 (138) = (S1c+S2c+S3c+S4c+S5c+S6c+S7c)/7, wobei die Signale Sxc dem normalisierten Autokorrelationssignal des Signals Sx aus S1, S2, S3, S4, S5, S6 und S7 entsprechen.

13. Computerprogrammprodukt, das vorzugsweise auf einem nichtflüchtigen Medium gespeichert ist und Anweisungen umfasst, die, wenn sie von einem Prozessor und/oder einem Computer ausgeführt werden, dazu führen, dass der Prozessor und/oder der Computer das entsprechende Verfahren (100) nach einem der vorhergehenden Ansprüche ausführt.

14. Messvorrichtung (10) für ein Photoplethysmogramm S(t) (111) eines Benutzers, die mit mindestens einer Datenverarbeitungseinheit verbunden ist, die mindestens einen nichtflüchtigen Speicher umfasst, der ein Computerprogrammprodukt nach dem vorhergehenden Anspruch umfasst, wobei die Messvorrichtung (10) mindestens einen Sensor (12) umfasst, der ausgelegt ist, ein Photoplethysmogramm zu messen.

15. Vorrichtung (10) nach dem vorhergehenden Anspruch, wobei der Sensor, der zum Messen eines Photoplethysmogramms ausgelegt ist, ein Sensor ist, der aus mindestens einem der folgenden ausgewählt wird: optischer Sensor (12), elektrischer Sensor, Radarsensor, Kraftsensor, Drucksensor, Vibrationssensor, Audiosensor, seismischer Sensor.

## Claims

1. A method (100) for determining the respiratory rate R of at least one user comprising at least the following steps implemented by at least one data processing unit comprising:
a. Acquisition of at least one photoplethysmogram S(t) of said user, the step of acquiring said photoplethysmogram S(t) comprising at least one of the following steps:
∘ Measurement, preferably at a frequency which is greater than or equal to 50Hz, of said at least one photoplethysmogram S(t) by a measuring device, preferably worn or intended to be worn by said at least one user, the device comprising at least one sensor (12) capable of measuring at least one photoplethysmogram S(t);
∘ Reception of said at least one photoplethysmogram S(t) from at least one database;
b. Estimate (120) of the heart rate Fc from said at least one photoplethysmogram S(t) acquired from said at least one user;
c. Obtaining (130) of at least three signals (S1, ...S7) which are distinct from each other, each obtained signal (S1, ...S7) being a function of said at least one acquired photoplethysmogram S(t);
d. Generation (138) of a signal S8 from said at least three obtained signals (S1, ..., S7);
e. Algorithmic processing (140) of each of the at least three obtained signals (S1, ..., S7) and of the signal S8 by at least two algorithms Ai and Aj, so as to obtain at least two estimated respiratory rates, respectively Rx.Ai and Rx.Aj, for each signal Sx processed respectively by the algorithm Ai and by the algorithm Aj, each signal Sx being taken from said at least three obtained signals (S1, ..., S7) and the signal S8; the at least two algorithms Ai and Aj being distinct from each other;
f. Calculation (150) of at least two intermediate respiratory rates rm and rn from the estimated respiratory rates (Rx.Ai, Rx.Aj);
g. Determination (160) of the respiratory rate R by calculating the median of said at least two intermediate respiratory rates rm and rn.

2. The method (100) according to the preceding claim comprising, before the step of estimating the heart rate Fc, a step of filtering said photoplethysmogram, preferably by a band-pass filter, so as to generate a first filtered photoplethysmogram.

3. The method (100) according to the preceding claim comprising, after the step of estimating the heart rate Fc, an additional step of filtering said first filtered photoplethysmogram between Fc/20 and 4Hz so as to generate a second filtered photoplethysmogram.

4. The method (100) according to the preceding claim, wherein said at least three obtained distinct signals (S1, ..., S7) are taken from at least the following signals S1, S2, S3, S4, S5, S6 and S7:
a. a signal S1 (131) obtained by:
∘ Application of a band-pass filter between Fc/20 and Fc/2 to the second filtered photoplethysmogram;
b. a signal S2 (132) obtained by:
∘ determination of the peaks of the beats of the upper envelope of the second filtered photoplethysmogram;
∘ then by filtration of the signal of the peaks of the beats of the upper envelope by a band-pass filter between Fc/20 and Fc/2.
c. a signal S3 (133) obtained by:
∘ determination of the valleys of the beats of the lower envelope of the second filtered photoplethysmogram;
∘ then by filtration of the signal of the valleys of the beats of the lower envelope by a band-pass filter between Fc/20 and Fc/2;
d. a signal S4 (134) obtained by:
∘ Generation of a pulse velocity wave VPG by performing a first order derivative of the photoplethysmogram S(t) relative to the time coordinate of said photoplethysmogram S (t) ;
∘ Determination of the positions Pi of the peaks of the pulse velocity wave VPG;
∘ Obtaining of a signal of variation of the inflection points by reporting values of said photoplethysmogram S(t) at the positions Pi;
∘ Oversampling of the inflection point variation signal to a predetermined frequency Fs;
∘ Band-pass filtration of the oversampled signal between the frequencies Fc/20 and Fc/2;
e. a signal S5 (135) obtained by:
∘ Location of the inflection points Pi in a systolic rise of a heartbeat of the user by identifying the position of the peaks of the first order derivative of the photoplethysmogram S(t) relative to the time coordinate of said photoplethysmogram S(t);
∘ Obtaining of a variation signal of the inflection points Pi by reporting values of time differences between Pi and Pi-1;
∘ Oversampling of this signal of intervals of the inflection points Pi to a predetermined frequency Fs;
∘ Band-pass filtration between the frequencies Fc/20 and Fc/2 of the oversampled signal;
f. a signal S6 (136) obtained by:
∘ determination of the amplitude variations of the second filtered photoplethysmogram;
∘ then by filtration with a band-pass filter between Fc/20 and Fc/2 of the amplitude variation signal;
g. a signal S7 (137) obtained by:
∘ determination of the valleys of the beats of the lower envelope of the second filtered photoplethysmogram;
∘ oversampling of the signal of the valleys of the beats of the lower envelope to a predetermined frequency Fs;
∘ First order derivation of the oversampled signal according to the time coordinate;
∘ then by filtration of the signal derived by a band-pass filter between Fc/20 and Fc/2.

5. The method (100) according to any one of the preceding claims, wherein the step of generating a signal S8 (138) from said at least three obtained signals (S1, ..., S7) comprises at least the following steps:
a. Calculation, for each signal among said at least three obtained signals (S1, ..., S7), of a normalised autocorrelation signal;
b. Calculation of the arithmetic mean of the normalised autocorrelation signals.

6. The method (100) according to any one of the preceding claims, wherein the at least two algorithms Ai and Aj which are distinct from each other are taken from at least the following algorithms A1, A2, A3 and A4:
a. an algorithm A1 (141) comprising at least the following steps applied to the signal Sx:
∘ Collection of the measurement points corresponding to a crossing of the abscissas by the considered signal, i.e. Sx(t)=0;
∘ Calculation of the average interval Tm between each of the abscissa crossing points;
∘ Calculation of Rx.A1 with Rx.A1 = 60/(2*Tm);
b. an algorithm A2 (142) comprising at least the following steps applied to the signal Sx:
∘ Collection of the measurement points corresponding to the peak of the ripples of the considered signal;
∘ Calculation of the average interval Ts between each of the points of the peaks;
∘ Calculation of Rx.A2 with Rx.A2 = 60/(Ts);
c. an algorithm A3 (143) comprising at least the following steps applied to the signal Sx:
∘ Determination of the correntropy spectral density by calculating the Fourier transform of the centred autocorrentropy of the considered signal;
o Extraction of Rx.A3 by determining the peak of the correntropy spectral density over a predetermined frequency range;
d. an algorithm A4 (144) comprising at least the following steps applied to the signal Sx:
∘ Calculation of the autocorrelation signal of the considered signal;
∘ Collection of the measurement points corresponding to the peak of the ripples of the autocorrelation signal of the considered signal;
∘ Calculation of the average interval Tas between each of the points of the peaks;
∘ Calculation of Rx.A4 with Rx.A4 = 60/(Tas).

7. The method (100) according to the preceding claim wherein the at least two intermediate respiratory rates rm and rn are taken from at least the following intermediate respiratory rates r1, r2, r3 and r4:
a. r1 (151) is equal to the median value of the estimated respiratory rates Rx.Ay of a signal Sx, Sx being taken from at least the obtained signals;
b. r2 (152) corresponds to the peak of the histogram formed by the estimated respiratory rates Rx.Ay of each considered signal Sx taken from the obtained signals processed through each considered algorithm Ay taken from A1 to A4;
c. r3 (153) is equal to the median value of the estimated respiratory rates Rx.A3 for each considered signal Sx taken from the obtained signals processed by the algorithm A3;
d. r4 (154) is equal to the median value of the estimated respiratory rates R8.Ay of the signal S8 processed through each considered algorithm Ay taken from A1 to A4.

8. The method (100) according to any one of the preceding claims, wherein the step of obtaining at least three signals (S1, ..., S7) which are distinct from each other comprises obtaining at least 4, preferably at least 5, advantageously at least 6 and advantageously the 7 distinct signals S1, S2, S3, S4, S5, S6 and S7.

9. The method (100) according to any one of the preceding claims in combination with claim 6, wherein the algorithmic processing step comprises the use of at least 3 and preferably the 4 distinct algorithms A1, A2, A3 and A4 applied to each of the obtained signals (S1, ..., S7) and the signal S8.

10. The method (100) according to the two preceding claims taken in combination, wherein r1 (151) = median (median ((R1.A1, R1.A2, R1.A3, R1.A4)), median ((R2.A1, R2.A2, R2.A3, R2.A4)), median ((R3.A1, R3.A2, R3.A3, R3.A4)), median ((R4.A1, R4.A2, R4.A3, R4.A4)), median ((R5.A1, R5.A2, R5.A3, R5.A4)), median ((R6.A1, R6.A2, R6.A3, R6.A4)), median ((R7.A1, R7.A2, R7.A3, R7.A4))).

11. The method (100) according to claims 7, 8 and 9 taken in combination:
- wherein r2 (152) corresponds to the peak of the histogram formed by the following values: R1.A1, R1.A2, R1.A3, R1.A4, R2.A1, R2.A2, R2.A3, R2.A4, R3.A1, R3.A2, R3.A3, R3.A4, R4.A1, R4.A2, R4.A3, R4.A4, R5.A1, R5.A2, R5.A3, R5.A4, R6.A1, R6.A2, R6.A3, R6.A4, R7.A1, R7.A2, R7.A3, R7.A4 and/or
- wherein r3 (153) = median (R1.A3, R2.A3, R3.A3, R4.A3, R5.A3, R6.A3, R7.A3).

12. The method (100) according to any one of the preceding claims taken in combination with claim 8, wherein S8 (138) = (S1c+S2c+S3c+S4c+S5c+S6c+S7c)/7, with the signals Sxc corresponding to the normalised autocorrelation signal of the signal Sx taken from S1, S2, S3, S4, S5, S6 and S7.

13. A computer program product, preferably recorded on a non-transitory medium, comprising instructions, which when performed by at least one of a processor and a computer, cause the at least one of the processor and the computer, executes the method (100) according to any one of the preceding claims.

14. A device for measuring(10) a photoplethysmogram S(t) (111) of a user connected to at least one data processing unit comprising at least one non-transitory memory comprising a computer program product according to the preceding claim, the measuring device (10) comprising at least one sensor (12) capable of measuring a photoplethysmogram.

15. The device (10) according to the preceding claim, wherein the sensor capable of measuring a photoplethysmogram is a sensor taken from at least: optical sensor (12), electrical sensor, radar sensor, force sensor, pressure sensor, vibration sensor, audio sensor, seismic sensor.
